# EUROPEAN PATENT SPECIFICATION

(11) **EP 3 288 391 B1**
(45) Date of publication and mention of the grant of the patent: **17.06.2020**
(21) Application number: 16728723.4
(22) Date of filing: 18.04.2016
(51) Int. Cl.: A23K 20/20, A23K 20/105, A23K 20/111, A23K 50/30, A23K 50/60, A61P 1/12

(54) **FEED SUPPLEMENT ADDITIVE**
FUTTERMITTELZUSATZSTOFF
ADDITIF DE COMPLÉMENT ALIMENTAIRE POUR ANIMAUX

(30) Priority: 28.04.2015 HU P1500195
(43) Date of publication of application: 07.03.2018
(73) Proprietor: Alpha- Vet Állatgyógyászati KFT., 8000 Székesfehérvár (HU)
(72) Inventor: MÓRÉ, Attila, 8000 Székesfehérvár (HU)
(74) Representative: Kovári, Zoltán
(86) International application number: PCT/HU2016/050013
(87) International publication number: WO 2016/174485

(56) References cited:
- AT-A1- 509 914
- N KUNAVUE ET AL: "Effects of Fulvic Acid and Probiotic on Growth Performance, Nutrient Digestibility, Blood Parameters and Immunity of Pigs", JOURNAL ANIM SCI ADV, vol. 2, no. 8, 1 January 2012 (2012-01-01) , pages 711-721, XP055285008,
- S W KIM ET AL: "Relative Availability of Iron in Mined Humic Substances for Weanling Pigs", ASIAN-AUST. J. ANIM. SCI., vol. 17, no. 9, 1 January 2004 (2004-01-01), pages 1266-1270, XP055296279,
- G M Hill ET AL: "Growth promotion effects and plasma changes from feeding high dietary concentrations of zinc and copper to weanling pigs (regional study).", J Animal Science, 1 January 2000 (2000-01-01), pages 1010-1016, XP055296397, Retrieved from the Internet: URL:https://www.animalsciencepublications. org/publications/jas/pdfs/78/4/1010 [retrieved on 2016-08-18]
- K.M.S. Islam . ET AL: "Humic Acid Substances in Animal Agriculture", Pakistan Journal of Nutrition, 1 March 2005 (2005-03-01), pages 126-134, XP055285010, DOI: 10.3923/pjn.2005.126.134 Retrieved from the Internet: URL:http://www.pjbs.org/pjnonline/fin295.p df [retrieved on 2016-08-18]

## Description

The object of the invention relates to a feed supplement additive containing humic acids, zinc and copper compounds for use in the prevention and/or treatment of diarrhoea diseases in suckling and weaned piglets, the production of the additive, as well as a feed containing such additive.

### The state of the art

Diarrhoea in suckling and weaned piglets is a worldwide problem in the pig industry. Dewey et al in their presentation at the London Swine Conference (2004, publication in person) highlighted that the occurrence of forms of diarrhoea caused by Escherichia coli is increasing, and that these infections cause significant economic losses all over the world. The greatest individual loss is expected to come from enteritis and diarrhoea (coli-enteritis) in weaning age piglets. A study was written in the past in the subject: Swine Health and Production- Volume 3, Number 3. 105-112 (1995).

Weaning in the life of pigs is almost the most critical production phase, and it represents huge physiological stress for the young animals. Due to the changed physical and social environment and due to the piglets receiving feed, piglet-diarrhoea is almost an everyday phenomenon in this production phase. Weaning diarrhoea may be caused by various obligates and facultative pathogens. Its appearance must also be counted on even in livestock populations that are viewed as excellent from the aspect of population health. According to Van Kempen et al (2014, Pig Progress News, knowledge & career 6 June 2014) it is precisely because of this that weaning diarrhoea is a "chicken or the egg" problem - do the piglets become infected with obligate pathogens and this is why they suffer from weaning diarrhoea, or is it because of the social and physical stress that they do not eat so much feed and therefore their immune system is weakened and so become infected with facultative pathogens?

It is especially favourable for the development of diarrhoea caused by Escherichia coli if the health condition of the sows is not appropriate, if the farm population is large, or if milk replacement piglet feed is used in an industrial-scale manner, and due to the large litters the piglets are cross-fostered between the ages of 1-14 days. It is precisely this tendency that may be observed in modern pig production facilities, including selection trends to increase litter size due to productivity and economics, and production trends endeavouring to save "biologically superfluous" piglets.

The significance of the subject is also confirmed by efforts aimed at the reduction of antimicrobial substances. Antimicrobial substances have been used in the global pig industry for more than 50 years in the feeding of weaned piglets - at subtherapeutic doses - due to the yield-increasing effects experienced. Most pigs raised in the USA receive antibiotics in their feed during the production cycle, but the same is also true in the case of European and Hungarian populations.

Present pig production systems are based on the widespread use of antimicrobial substances, as numerous active substances are distributed for the treatment of coli-diarrhoea. The disadvantages of antibiotics administered for this purpose are the development of resistance and the formation of substance traces in foodstuffs. On the basis of the basic principles of the European Medicines Agency, Committee for Medicinal Products for Veterinary Use, the use of antibacterial active agents will be significantly limited in the future (EMA/CVMP/287420/2010, CVMP Strategy on Antimicrobials 2011-2015). In accordance with this, various antibiotic active substances may only be administered to diseased individuals in the future; their use for the prevention of infectious diseases is not recommended.

Almost all companies involved in pig breeding have worked out management, technology, animal health and feed strategies for the prevention of diarrhoea diseases in suckling and weaned piglets. In spite of this - as a result of the selection and production trends - diarrhoea currently causes the greatest amount of financial loss, production drops, and outages in the piglet raising phase.

Biksi et al identified the following diarrhoea-causing pathogens in the faeces and environments of suckling and newly weaned piglets on Hungarian pig farms (Őstermelő - Gazdálkodók lapja [Producers and Farmers Journal] 2013, issue 6): Escherichia coli, Clostridium perfringens A, Clostridium perfringens C, Clostridium difficile, Enterococcus durans.

Apart from the listed bacteria, the following virus-caused diarrhoeas occur on Hungarian pig farms: swine transmissible gastroenteritis (TGE), diarrhoea caused by rotaviruses, diarrhoea caused by coronaviruses, Porcine reproductive and Respiratory Syndrome (PRRS). Apart from the bacteria and viruses, coccidiosis (Isospora suis) also frequently causes diarrhoea in suckling and newly weaned piglets.

On the basis of the studies performed by Biksi et al (2012, see above) various pathogens, as main pathogen, cause the diarrhoea at different ages. The distribution over time of the infections was the following on the Hungarian farms participating in the study:

**Table 1:**

| Age | Pathogen |
|---|---|
| Generally during the first 14 days | Echerichia coli |
| Generally during the first 14 days | Clostridium spp. |
| Generally between the 8^{th} and 21^{st} day | Isospora suis |
| Generally during the first 14 days | Transmissible gastroenteritis (TGE) |
| Generally during the first 14 days | Porcine Epidemic Diarrhoea (PED) |
| Generally between the 8^{th} and 21^{st} day | Rotaviruses |
| Generally between the 8^{th} and 21^{st} day | Coronaviruses |
| Continuously during suckling | PRRS |

According to the investigations performed by Dr Böő (Producers and Farmers Journal 2013, issue 6) apart from the above pathogens, diarrhoea in newly weaned piglets may also be caused by Post Weaning Multisystemic Wasting Disease (PMWS). This disease was first described in Hungary in 1999. Extensive studies performed since then have determined that it is caused by the Porcine Circovirus 2 (PCV2).

From the above it can be seen that focussing on the main pathogens, preventing diarrhoea in suckling and newly weaned piglets demands very wide-ranging population health strategies. In most pig farms the sow population is inoculated against Escherichia coli and Clostridium perfringens C. There are no vaccines available against Clostridium perfringens A. It must be noted here that both E. coli and Clostridium spp. are natural components of pig microbiota, even in natural conditions they are present in the digestive tract of sows and its vicinity. According to Juan Luis Ubeda (2014, NNPD phenomenon in the modern pig breeding - presentation, MAGAPOR Technical Seminal 2014, Zaragoza, Sow management Round Table Session) this natural microbiota is changed by vaccination, which fact may make individuals susceptible to secondary infections. Due to this, increased emphasis is being placed in the international literature and conferences on communicating that the use of probiotics and natural immune stimulants as feed supplements is essential beside the vaccinations. Apart from the use of vaccines, feed medication with antimicrobial substances - tylosin, tiamulin, tetracycline - is used at affected pig farms against Clostridium spp. Piglets are treated individually on pig farms according to a protocol for the prevention of coccidiosis caused by Isospora suis.

Therefore, appropriate active substances, antimicrobial agents are available in the case of the known pathogens for the prevention of piglet diarrhoea.

Nevertheless, the significance of the problem is confirmed by the fact that new diarrhoea diseases of unknown origin occur almost every year, against which the known active agents and protocols are almost completely ineffective. For example, in 2012 Neonatal Piglet Disease (NNPD) was merely just mentioned, while by 2014 it was a subject of professional discussion and debate on the MAGAPOR Technical Seminar. According to experience, the presumably polyfactorial disease of unknown cause occurs in populations with the greatest genetic value and the highest production level and causes epidemic-like diarrhoea diseases in 3-4 day old piglets. According to investigations performed by Ubeda (2014, see above) it occurs with greater frequency in the piglets of sows suffering from PPDS (Postpartum Dysgalactia Syndrome). The effect of the antibiotics used to treat the condition is not clear. In most cases the development of PPDS is preceded by extended farrowing - lasting for more that 3 hours. The farrowing of modern pigs lasts longer than 3 hours in 60% of the population as a result of selection aimed at overmuscularity and large litters, which leads to the conclusion that the frequency of NNPD will increase. The virus causing Porcine Epidemic Diarrhoea (PED) is also new; it is viewed as a pathogen causing diarrhoea. Its significance is underlined by the fact that 3 new strains have been identified since its occurrence in April 2013. By May 2014 46% of the USA pig population was affected by the disease. The pathogen infects both sows and suckling and newly weaned piglets. However, the greatest damage is caused when the newly weaned piglets become infected. The antimicrobial substances known of to date have proved to be completely ineffective. Several international companies have started to develop a vaccine, however, there is no vaccine currently commercially available against the pathogen.

International companies started extensive research work in the interest of finding possible alternatives; they are continuously testing enzymes and phytogenic additives, as possible alternatives to the antibiotics used during weaning. It may be determined that natural immune stimulation will become a key factor in the feeding of newly weaned piglets in the near future. The animal health quality and feed intake of piglets will improve with the appropriate immune stimulants.

The above again support the importance of the use of natural immune stimulants in modern pig breeding systems. Due to the concentration of population sizes, the selection trends, the increasing feeding challenges, and the continuously mutating and recombining pathogenic microorganisms, the preventive, process-integrated application of natural immune stimulants will become essential in the piglet-raising phase. Currently prebiotic and probiotic substances, medicinal herbs, metal ions and drinking water acidifiers are used, but effectiveness is not sufficiently proven in the case of numerous products.

Apart from using antimicrobial substances to protect against weaning diarrhoea, Hungarian and European pig farms use 2000-3000 ppm zinc oxide feed medication as part of their process.

The yield-increasing effect of zinc oxide widely used for the prevention of weaning diarrhoea comes from the zinc. Zinc is a structural or functional component of 300 known enzymes and participates in at least this many biochemical processes. These processes are more significant in the growing organism, and so this is why a yield-increasing effect is experienced in the young, newly weaned piglets. According to Poulsen and Carlson (2008, Trace elements in animal production systems, 151-160, Wageningen Academic Publishers) zinc catalyses approx. 200 transcription factors, and due to this it is an element of fundamental importance in quickly dividing, growing and proliferating cells, such as the cells and immune system of newly weaned piglets.

However, the SCAN scientific committee opinion of 2003 questioned the effect of zinc to improve growth, according to the opinions of several specialists in the subject, the use of a pharmacological amount of zinc oxide in feed has a positive effect on weaned piglet production because at this concentration the zinc oxide promotes the increase in diversity of coliform microbes in the small intestine. In a diverse microbiota Echerichia coli, which causes enterotoxemia, is unable to proliferate to the extent that would cause diarrhoea. The investigations performed by Ubeda (2014, see above) also support this. According to this the effect of the vaccine used against Escherichia coli actually causes a change in the microbiota of sows and, through the sows, in the microbiota of the piglets as well. However, the microbiota changed due to the vaccine does not make it possible for the newly weaned piglets to achieve their potential yield, this is why zinc oxide is seen to increase yield.

Beside the known and experienced preventive effects, the use of zinc oxide as a medication causes concerns from both the points of view of environmental protection and physiology.

The accumulation of zinc in the soil from pig manure was already a worrying problem in the 1970s, however zinc oxide medication of 2000-3000 ppm became a standard part of the process from the 1990s in the case of newly weaned piglets. According to the calculations performed by Untea et al (Journal of Trace Elements in Medicine and Biology 25S(2011)S35-40) if the accumulation of zinc in the soil from pig manure continues at the current rate, the topsoil will be completely unsuitable for agricultural production in approximately 50 years. At the currently used levels of pharmacological zinc oxide, 1.5 kg of zinc per hectare gets into the soil in one year.

The reduction of zinc oxide concentration used in pharmacological amounts in weaned piglet feed is viewed as an important objective to be achieved by those dealing with animal feed and pig breeding. Research has been carried out in this field from several approaches. One possible alternative was thought to have been discovered in zinc proteinates. Rinker et al (Journal of Animal Science, 2005, 83:2762-2774) compared the animal health and yield-increasing effect of zinc oxide to zinc methionine and found that the yield-increasing was the same in clinically healthy piglets. The amount of zinc excreted with the faeces was lower in the case of the zinc methionine medication. Certified by physiological studies, the zinc oxide is stored in the piglets' bodies during supplementation administered for 9-10 days, then after the zinc stores have been filled, zinc starts to be excreted. In studies performed by Rinker (2005, see above) in the case of zinc oxide administered in pharmacological amounts (3000 ppm) the zinc balance was negative already on the 13^{th} day following weaning, in other words the piglets excreted more zinc with the faeces than they consumed in their feed!

Another potential pathway for reducing zinc oxide use may be the use of phytogenic feed supplements. Several international companies dealing with animal feed are performing extensive research work in this field. The precise effect mechanisms, appropriate biological formulas cannot yet be viewed as being clear. However, in most cases it was possible to reduce the pharmacological zinc oxide level used in the piglets' feed and add phytogenic additives to achieve the appropriate piglet yield (Untea et al, see above), BIOMIN Gmbh (2014). Several phytogenic additives are now commercially available, however, their widespread integration into the process is not expected for the time being due to the high market price.

Apart from the above, humic acids are also used to supplement piglet feed. According to Kunavue and Lien (J Anim Sci Adv 2012, 2(8): 711-721) the energy utilisation, carbohydrate digestion, phosphorous utilisation and the plasma immunoglobulin concentration of piglets improved with humic acid supplements. The natural production indicators of newly weaned piglets complied with the expectations, as compared to the commercially available antimicrobial substance selected as a control, there was no significant difference in the weight gain of the piglets due to the effect of the humic acid supplement.

The therapeutic, anti-inflammatory use of humic acids in combination with metal ions is confirmed by the described and certified effects given in international literature. Riede et al (Virchows Archiv B December 1991, Volume 60, Issue 1, pp 27-34) proved that it was possible to increase the natural activity of neutrophilic granulocytes with low molecular weight humic acids. Low molecular weight humic acids activate neutrophilic granulocytes as a result of their chemical structure. Van Rensburg et al (Inflammation, 2004 Jun; 28(3):169-74.) proved that the anti-inflammatory effectiveness of natural humic acids was similar to prednisolone in rat models. The humic acids reduced the hypersensitivity of the inflamed tissues. It was not possible to identify toxicity even after a month of treatment. Constance et al (Inflammation, Vol. 32, No 4, August 2009 (# 2009)) proved that humic acids increase lymphocyte proliferation, both on the classical and alternative reaction path. During this the structure of red blood cells was not influenced. On the basis of the results, humic acids were designated as potential anti-inflammation agents. Naude et al (Inflammation, 2009 Aug; 32(4):270-6) proved the inflammation-reducing effect of humic acids not only with immunological parameters but also with the clinical manifestations of the oedemas. Humic acid treatment was more effective than indomethacin treatment. Islam et al (Pakistan Journal of Nutrition 4(3). 126-134, 2005.) proved that the stress reaction is less intense in the presence of humic acids, Cannon's fight-or-flight reaction occurs later, because less cortisol is produced. This was certified in Spanish fighting bulls as well as in transported swine and cattle.

There is much data available in the literature about the antiviral effect of humic acids. Systematic research was started in this field when it accidentally turned out that certain peat preparations successfully cure foot-and-mouth disease. In further research it was proven that humic acids inhibit the adsorption and synthesis of numerous RNA and DNA type viruses. The conclusion that can be made from the studies is that humic acid primarily inhibits virus adsorption. During their investigations Klöcking et al were able to suppress the reproduction of the following viruses using anti-haemophilic factor produced from natural humic acids: Human Immune-deficiency Virus (HIV), Bovine Herpes Virus (BHV), Bovine Virus Diarrhoea Virus (BVDV), Hepatitis A Virus, Muréna Encephalomyocarditis Virus (EMCV), Porcine Parvovirus (PPV)

It was also found that the humic acids bind at the positively charged places of the protein coat of viruses and so prevent them binding to tissues.

There is also information available about the antibacterial effect of humic acids. The antiseptic effect of peat preparations has been known for along time, there is even knowledge relating to this from Ancient Egyptian times. One of the most tried and tested methods for preventing infection of wounds in the 1^{st} World War was peat dressing.

As compared to its antiviral effect, the antibacterial effect of humic acids is much more dose-dependent. The inhibiting dose of humic acids is orders of magnitude smaller than that of antibiotics. The humic acids primarily influence the catalytic metabolism processes of microbes. An expressly damaging effect can be observed against bacteria and virus particles. Humic acids exert an indirect protective effect against damaging microbes by reacting with the large molecule cell protein toxins and with the ion bonds of pathogenic microbes, through this they prevent their absorption through the mucous membrane. The protective effect affects useful microbes positively, as their metabolisms may get damaged by the exogenous toxins produced by the pathogens. This commensalistic microbe damage, and so production reduction of the farm can be prevented with humic acids.

Investigations have been carried out regarding the humic acid sensitivity of numerous microbes, among them the following were determined to be sensitive to humic acid: Staphylococcus epidermidis, Staphylococcus aureus, Streptococcus pyogenes, Salmonella typhimurium, Proteus vulgaris, Enterobacter cloacae, Pseudomonas aeruginosa.

Humic acids have a proven inhibitor effect on the reproduction of the following microorganisms: Candida albicans, Enterococcus cloacae, Staphylococcus epidermidis, Staphylococcus pyogenes.

According to Islam et al (see above) the use of humic acids in livestock farming is justified from several points of view. Several authors have described a proven growth promoting effect in the case of broiler chickens, turkeys and dairy calves (Teravita Inc., http://www.teravita.com/humates/chapter9.htm), and in the case of dairy calves an increase in plasma immunoglobulin concentration was also observable, which was indicated in the case of pigs previously. The frequency of udder inflammation drops due to the known antimicrobial effects of humic acids.

Pisarikova et al, Humintech Inc. (2004) proved that humic acids promote the absorption and utilisation of minerals in feed.

Kühnert et al (Deutsche Tierärztl, Wschr, 96: 3-10) proved that humic acids are capable of establishing a protective bio-film layer on the mucous membrane of the small intestines due to their macrocolloid structure. With this the stomach and the small intestines are protected from ulcerous acid burns, and from absorption of toxic metabolites and bacterial endotoxins. This protective bio-film layer also prevents water leaving the intestine cells.

In their investigations Ji et al (J. Anim. Sci. 2006, 84:2482-2490) achieved a significantly greater daily weight increase in pigs consuming feed supplemented with humic acids due to the beneficial effects of humic acids, they had more favourable slaughterhouse yield, and the amount of ammonia emitted with the pig manure also dropped due to the effect of the humic acids supplement.

Sung Wo Kim's research team (http://www.a-1organiclawns.com/1/a1/files/liveearthproposaliigrowth studyinpigs.pdf) also succeeded in proving the favourable effects exerted on slaughterhouse parameters. During their studies they experienced that the pigs receiving the humic acid supplement had fewer pathological liver and kidney conditions. A further relevant background art is the following publication: S W Kim et al.: "Relative Availability of Iron in Mined Humic Substances for Weanling Pigs", Asian-Aust. J. Anim. Sci., vol. 17, mo. 9, 1 January 2004, pages 1266-1270.

Austrian patent application AT 509 914 A1 is also a relevant background art.

Summarising the above, one of the greatest source of loss in pig breeding is caused by enteritis and diarrhoea (coli-enteritis) in weaning-age piglets, which is fought against with antibiotic treatment, vaccination, natural immune stimulants or phytogenic feed supplements and, the most widespread, zinc oxide feed medication. The disadvantage of antibiotic treatment is the development of resistance, and the formation of foodstuff substance traces. Antibiotic treatment is not compatible with ecological (organic) production, not only because of the developed microbial resistance and the existing human health risks. Vaccination changes the natural microbiota, which may make the animals susceptible to secondary infections. Furthermore, it is not sure if these are effective against new pathogens. For example NNPD occurs with greater frequency in the piglets of sows suffering from PPDS (Post Partum Disgalactia Syndrome). The effect of antibiotics is not clear in the treatment of the condition. The PED virus is also new; it is viewed as a diarrhoea-causing pathogen. The antimicrobial substances know to date have proven to be completely ineffective in its treatment. What is more, due to the above disadvantages, legislative restrictions are reducing the use of antibiotics, and, if possible, they should be left out of the feed of newly weaned piglets. The use of natural immune stimulants is important, but in the case of numerous products, effectiveness is not sufficiently supported.

The precise effect mechanism of phytogenic feed supplements, and the appropriate biological formulas are not yet viewed as being clear. Several phytogenic additives are already commercially available, however, their widespread use in the production process is not expected due to their high market price.

The use of zinc oxide feed medication is not at all favourable from the point of view of environmental protection. If the accumulation of zinc in the soil from pig manure continues at the present rate, the topsoil will be completely unsuitable for agricultural production in approximately 50 years. With the currently used pharmacological zinc oxide levels, 1.5 kg of zinc gets into one hectare topsoil every year. The reduction of the concentration of zinc oxide used in pharmacological amounts is, due to this, treated as an important objective to achieve in newly weaned piglet feed for those dealing with feed production and pig breeding.

Therefore, we set an objective of producing a feed supplement mixture that is suitable for replacing or changing zinc oxide feed medication so that the above environmental protection and animal health problems do not occur and, by using cheap raw materials, of replacing antibiotic treatment and vaccination in the case of weaning clinically healthy piglets.

The objective was achieved by producing a feed supplement preparation containing humic acids, zinc and copper compounds for use in the prevention and/or supplementary treatment of diarrhoea in suckling and newly weaned piglets. As it was surprising to experience that if zinc, copper and humic acids are used in combination as a feed supplement, then the zinc intake can be significantly reduced as compared to zinc oxide medication, which significantly reduces the amount of heavy metal pollution originating from the excretion of zinc. Also, the active agents of the invention, zinc, copper and humic acids, are materials with respect to which resistance is not expected to develop.

Viewed more closely, the object of the invention is a feed supplement preparation containing humic acids, zinc and copper compounds for use in the prevention and/or supplementary treatment of diarrhoea diseases in suckling and newly weaned piglets.

It was surprising to experience that if the feed supplement preparation according to the invention containing humic acids, zinc and copper compounds or a feed containing such a supplement is used for the prevention and supplementary treatment of diarrhoea diseases in suckling and newly weaned piglets, then as compared to zinc oxide treatment, zinc intake can be reduced by an order of magnitude. The frequency and severity of piglet diarrhoea in compared populations in large intensive farms was not significantly different when treated with the preparation according to the invention or zinc oxide. What is more, in the later piglet-raising phase in the case of the use of the feed supplement according to the invention the piglets achieved a greater daily weight increase than a population receiving zinc oxide medication when there was no difference in the health condition of the two groups. The zinc content with the preparation according to the invention was 130 ppm, while the control preparation containing zinc oxide (Zintestin Forte) was used at the pharmacological concentration of 2600 ppm as recommended by the manufacturer. Therefore, by using the preparation according to the invention the desired objective was achieved even by reducing zinc intake to one twentieth, i.e. prevention and supplementary treatment of diarrhoea and protection of the environment against unnecessary heavy metal pollution.

So the feed supplement preparation according to the invention may be preferably used for the prevention and/or supplementary treatment of diarrhoea diseases in suckling and newly weaned piglets caused by physiological stress and/or pathogenic microorganisms.

Therefore, the preparation according to the invention is also suitable for use in the prevention and/or supplementary treatment of diarrhoea diseases the pathogens of which are Escherichia coli, Clostridium perfringens A, Clostridium perfringens C, Clostridium difficile, Enterococcus durans, Transmissible Gastroenteritis (TGE), rotaviruses, coronaviruses, Isospora suis, Clostridium spp., the Porcine Epidemic Diarrhoea (PED) virus, Porcine Circovirus 2 (PCV2), bacteria or other microorganisms. The preparation according to the invention is also suitable for use in the prevention of coli-enteritis, Transmissible Gastroenteritis, porcine reproductive and respiratory syndrome (PRRS), coccidiosis, post weaning multisystemic wasting disease, and neonatal piglet disease (NNPD).

Viewed in more detail, an especially preferable form of the invention for the above use is a feed supplement preparation in which the mass ratio of the humic acids (HS), the zinc (Zn) and copper (Cu) calculated using elemental zinc and copper is between 100 (HS) :2-10 (Zn) : 1-10 (Cu), preferably 100(HS):2-6 (Zn): 2.5-7 (Cu), and most preferably 100(HS):2.5-5 (Zn): 3-6 (Cu).

As the source of humic acid, copper and zinc, the feed supplement preparation may contain any material containing humic acid, copper and zinc compounds or materials, mixtures containing these that are generally used or may be used in livestock breeding. Those compounds may be used in livestock breeding that at therapeutic doses do not damage the animal's development, that do not reduce the animal's lifetime and their therapeutic benefit does not outweigh the damage originating from any possible adverse effects. As the source of humic acid, the feed supplement contains fulvic acid, peat or lignite or a mixture of these, more preferably fulvic acid and peat containing at least 40% humic acid or a mixture of these, as the source of zinc, organic or inorganic zinc salts, complexes, the hydrates of these or zinc dioxide, as an organic salt or complex preferably zinc humate, zinc acetate, zinc citrate, as an inorganic salt zinc sulphate, zinc chloride, preferably zinc sulphate monohydrate, as a source of copper organic or inorganic copper salts, complexes, or hydrates of these, preferably copper sulphate.

It may contain any zinc or copper compound, salt, complex or hydrates of these as the source of zinc and copper. As a source of zinc it preferably contains organic or inorganic zinc salts, or the hydrates or complexes of these, or zinc dioxide, and as a source of copper it contains organic or inorganic copper salts, complexes or the hydrates of these. Even more preferably the preparation contains zinc humates, zinc acetate, zinc citrate, as an inorganic salt zinc sulphate, zinc chloride, most preferably zinc sulphate monohydrate, and copper sulphate, mono or pentahydrate as the copper compound.

The feed supplement preparation containing humic acids, zinc and copper compounds according to the invention for the prevention and/or supplementary treatment of suckling and newly weaned piglets contains 60-800 ppm zinc, preferably 60-260 ppm zinc, even more preferably 90-190 ppm zinc, most preferably 100-160 ppm zinc for use at pharmacological doses.

The above feed supplement preparation containing humic acids, zinc and copper compounds may be produced by mixing the humic acids or materials containing humic acids, preferably peat or lignite with copper and zinc compounds as well as, in a given case, with further auxiliary materials. The procedure is performed by using the humic acids, the zinc compounds and copper compounds at the following ratios calculated for elemental zinc and copper: 100(HS):2-10 (Zn): 1-10 (Cu), preferably 100(HS):2-6 (Zn): 2.5-7 (Cu), most preferably 100(HS):2.5-5 (Zn): 3-6 (Cu). As the source of humic acid, copper and zinc, any material containing humic acid, copper and zinc compounds or materials, mixtures containing these may be used for the production of the feed supplement preparation that are generally used or may be used in livestock breeding.

As the source of humic acid, the feed supplement contains fulvic acid, peat or lignite, preferably fulvic acid, peat or a mixture of these, most preferably a mixture of fulvic acid and peat containing at least 40% humic acid. Any zinc or copper compound, salt, complex or their hydrates may be used as the source of zinc and copper. Preferably organic or inorganic zinc salts or their hydrates, complexes or zinc dioxide may be used as the source of zinc, and organic or inorganic copper salts, complexes or their hydrates may be used as the source of copper. Even more preferably the preparation contains zinc humate, zinc acetate, zinc citrate, as an inorganic salt zinc sulphate, zinc chloride, most preferably zinc sulphate monohydrate, and, as a source of copper, copper sulphate, or mono or pentahydrate.

Most preferably the process includes measuring 100 mass parts of peat containing at least 40% humic acid, 4-6 mass parts of zinc sulphate, calculated for elemental zinc, preferably zinc sulphate monohydrate and 1-10 mass parts of copper sulphate, calculated for elemental copper, preferably copper sulphate monohydrate and 40-60 mass parts of powdered fulvic acid containing 70% fulvic acid into a mixing device and homogenising them.

However, the process may also involve pouring solutions of the zinc compound, preferably zinc sulphate, and the copper compound, preferably copper sulphate, or a mixture of their solutions into the mixture of peat and fulvic acid during mixing, or these are sprayed onto the powder mixture, then if necessary the wet powder is dried or made into a suspension by adding further auxiliary materials. The production of the suspension may take place with the methods used in agriculture for the production of plant protection leaf manures and by adding the necessary auxiliary materials. The selection of the method, the materials and the equipment belongs to the general knowledge of a person skilled in the art.

Both the powder mixtures and the suspension may be used by mixing them into the feed at the appropriate ratio, or by spraying the suspension onto the feed.

Therefore, the preparation according to the invention is a feed supplement additive containing humic acids, zinc and copper compounds. If necessary the feed supplement additive according to the invention also contains further auxiliary materials apart from the active substances. Preferably the mass ratio of the humic acids, the zinc and copper, calculated for elemental zinc and copper, is between 100(HS):2-10 (Zn): 1-10 (Cu), preferably 100(HS):2-6 (Zn): 2.5-7 (Cu), most preferably 100(HS):2.5-5 (Zn): 3-6 (Cu).

The aforementioned materials may be used as the sources of humic acids, zinc and copper.

The effect of the preparation according to the invention was examined in experiments performed on the pilot plant and large production scales. The composition of the feed supplement preparation according to the invention used in the experiments was the following:

| Component: | |
|---|---|
| Peat (with min. 40% humic acid content) | 59.8-60.6 m/m% |

| | |
|---|---|
| Powdered fulvic acid with 70% fulvic acid content | 29.9-30.3 m/m% |
| Crystalline Zn-sulphate monohydrate | 3.4-4.0 m/m% |
| Crystalline copper-sulphate monohydrate | 5.7-6.3 m/m% |

The preparation was produced by measuring the components into a mixing device, then mixing and homogenising them. The preparation was given to the piglets in the feed.

In the pilot plant experiment the clinical efficacy of the humic acid feed supplement was examined in the prevention of artificially induced Escherichia coli diarrhoea in weaned piglets. The results obtained were compared with the zinc oxide medication method used in practice. The piglets taking part in the experiment received the aforementioned additives mixed in their feed for four weeks. On the 5^{th} day of feeding the piglets were infected on one occasion with a mixture of highly pathogenic E. coli strains isolated from clinical cases. The clinical symptoms resulting from the infection, their severity, the weight gain of the animals and the feed intake of the groups were studied. At the end of the study, the differences between the various treatment groups were compared. During the study the piglets in the experiment were kept in a separate enclosure, in a conventional way. The animals were kept in groups of 10. Lighting was maintained for 14 hours, which was partially natural and partially artificial (neon), the temperature was 15-25 °C, the relative humidity was (RAH) between 30-65%. Feed and drinking water was provided ad *libitum* during the entire duration of the experiment. The quality of the feed was commercial quality coarse prestarter feed.

During the term of the experiment 5 groups of 10 piglets each were established according to treatment and infection (table 2). The grouping of the individuals was made on the basis of bodyweight, in such a way that when the experiment was started the average weight of the groups was nearly the same.

**Table 2**

| **Treatment** | **Infection** | **Group name** | **Group number** |
|---|---|---|---|
| none | none | Control | Group 1 |
| none | infected | Infected control | Group 2 |
| 1% humic acid product mixed into the feed for 4 weeks | infected | Humic | Group 3 |
| 2800 ppm zinc ion mixed into the feed for 4 weeks | infected | Zinc oxide | Group 4 |
| The use of the feed supplement according to the invention (1% humic acid product + 130 ppm zinc-ion + 150 ppm copper) mixed into the feed for 4 weeks | infected | Humic-zinc-copper | Group 5 |

The experiment groups were fed with the various preparations for 4 weeks in such a way that they were mixed in advance into the feed. Feed intake was measured per group during the experiment and the average weight per piglet was recorded.

The data obtained during the experiment - feed intake, bodyweight change, clinical symptoms and their severity - were collated and assessed using statistics tests.

From the third week the greatest feed intake was observed in the humic group (group 3). During the entire period of the study the humic acid group supplemented with microelements (group 5) proved to have the lowest standard deviation value, which indicates that this group was the most homogenous.

The greatest standard deviation was found in the zinc oxide group (group 4), which may be due to the unreliable absorption of the zinc oxide. This partial result supported the hypothesis that the yield-increasing effect is not exerted directly by the zinc oxide but by influencing the microbiota. This was confirmed by the weight changes in the piglets after infection, where it was found that following artificial infection the piglets consuming feed medicated with zinc oxide (group 4) also lost weight, almost to the same extent as the group receiving the humic acid supplement (group 3). At the same time, due to the effect of the humic acid treatment supplemented with microelements according to the invention (group 5), during the week following infection the loss of weight in the piglets was just 50% of weight loss in the zinc dioxide group (group 4), and at the end of the experiment the weight of the piglets receiving humic acid treatment supplemented with microelements according to the invention (group 5) was the same as the weight of the piglets receiving zinc oxide treatment. In other words, with respect to the so-called yield-increasing effect, the humic acid product supplemented with microelements according to the invention was equivalent to the currently used zinc oxide.

The clinical symptoms of the piglets were also examined during the experiment. The animals in the infected groups had varying degrees (severity) of diarrhoea depending on the feed additive given. The most severe symptoms were observed in the infected piglets that did not receive treatment (group 2, infected control), while during this period the piglets in the control group (group 1) were without any symptoms all the way through the experiment. (It should be noted that in week 4 of the feeding experiment a number of individuals in the control group had mild diarrhoea.)

In the case of the groups receiving the various treatments, the number of seriously ill piglets was less on average, and the symptoms (listlessness, lack of appetite) were found to be milder as compared to the infected control group. The piglets in the humic group (group 3) had more severe symptoms overall and the duration of the diarrhoea was longer, while the individuals in the zinc oxide group (group 4) only had mild symptoms, and the symptoms disappeared sooner. The values of the piglets treated with the preparation according to the invention (group 5) fell between those of the previous two groups.

From the point of view of clinical efficacy, with regard to the number of diarrhoea days, the humic acid product supplemented with microelements according to the invention was hardly 10% less effective than the currently used zinc oxide.
With respect to the severity of diarrhoea, the effect of the humic acid feed supplement with microelements according to the invention was just 4% weaker than zinc oxide when compared to the infected control group. However, the severity of the diarrhoea is a subjective indicator, judging it is not uniform among livestock breeders and vets. This is shown by that at the end of the experiment the greatest weight gain was in the group receiving humic acid supplemented with microelements according to the invention (group 5). This is deemed important because during the nursery period it is not the severity of the diarrhoea that is decisive but the weight of the piglets when they go to the post-weaning phase. Production results show - 250 g bodyweight difference in the case of 8-week-old piglets - that the use of the feed supplement containing humic acid, zinc and copper is preferable to zinc oxide. In spite of the diarrhoea symptoms lasting two days more, 8-week-old piglets were produced that had a greater weight due to the effect of the humic acid and microelement treatment according to the invention.

It was also possible to prove the advantage of the preparation according to the invention over zinc oxide medication in large-scale production.

The experiment involved 74 experiment and 53 control piglets of mixed sex aged from 10 days to 55 days, which were from a Hungarian Large White x Hungarian Landrace population.

The experiment piglets were individually measured during suckling per litter at the ages of 10 and 28 days. The amount of feed intake during this period was measured per litter.

Weaning took place at an average of 32 days. The piglets were placed in post-weaning batteries at equal sex ratio. When the piglets were moved care was taken to ensure that a maximum of 5 piglets from other sows were placed in the same pen, which was carried out to model actual large-scale production conditions. 10 repetitions were performed for the experiment group and 9 repetitions for the control group. The piglets were individually weighed on the day of weaning (32 days old) and after 23 days of experiment feeding (55 days old).

Experiment feeds: From 10 days of age until the completion of the experiment the piglets were fed starter piglet feed. The control group of piglets was fed with the starter piglet feed used at the farm, supplemented with Zintestin Forte premix containing zinc oxide. The piglets in the experiment group received the same feed, which, instead of the Zintestin Forte premix containing zinc oxide, was supplemented with the humic acid additive with microelements according to the invention. The animals could feed ad libitum from a self-feeder and drink from bite ball valves.

The performance indicators of the piglets in the suckling piglet phase were as follows:

**Table 3**

| **Daily weight gain** | **Total weight gained** | **28 day weight** | **10 day weight** | |
|---|---|---|---|---|
| 195.1 g/day | 3.58 kg | 6.50 kg | 3.16 kg | **Control** |
| 182.0 g/day | 3.42 kg | 6.12 kg | 2.99 kg | **Treated** |
| -13.1 g/day | -0.16 kg | -0.38 kg | -0.17 kg | **Difference compared to control** |

The average daily weight gain was 8% greater in the control group, the difference is not significant.

During the suckling piglet phase the feed supplement according to the invention did not provide the yield-increasing effect that the zinc oxide medication (Zintestin Forte) did. The suckling piglets eat the preparation according to the invention and do not reject it. The preparation does not influence the intake of the prestarter piglet feed by the suckling piglets in the farrowing pen.

When moving the piglets after weaning the equal sex ratio was set for the two groups, and the runt piglets were taken out of the experiment. The control group piglets were placed in 9 pens and the treated piglets were placed in 10 pens. The piglet rearing experiment included 39 piglets in the control group and 50 piglets in the experiment group.

The performance indicators of the piglets in the post-weaning phase were as follows:

**Table 4**

| **Daily weight gain** | **Total weight gained** | **Closin g weight** | **Initial weight** | |
|---|---|---|---|---|
| 252.39 g/day | 6.06 kg | 14.06 kg | 8.00 kg | **Control** |
| 306.0 g/day | 7.34 kg | 15.12 kg | 7.78 kg | **Treated** |
| +53.61 g/day | +1.28 kg | + 1.06 kg | -0.22 | **Difference compared to control** |

The duration of the experiment was 23 days, from weaning (average age of 32 days) until the piglets reached the average age of 55 days. The daily weight gain, as opposed to the period before weaning, was 12% greater in the experiment group.

In other words, in the post-weaning phase the preparation according to the invention had a clearly positive effect on the growing vigour of the weaned piglets. A greater yield-increasing effect was achieved with the supplement according to the invention than with the currently used zinc oxide. This verified the yield-increasing effect experienced in the pilot-scale experiment and confirmed that the humic acid feed supplement with microelements according to the invention is a yield-increasing product equivalent to or more favourable than zinc oxide.

During the suckling phase 19% of the control group and 61% of the animals treated with the preparation according to the invention had mild, non-repeating diarrhoea. It was observed that in the group treated with the preparation according to the invention diarrhoea mainly occurred in the suckling piglets in the farrowing pen after they reached 18 days of age, which may suggest that the composition of the sow's milk changes due to the reactivated ovaries and the reoccurring ovarian hormones, and that it is this that generates the diarrhoea in the more sensitive piglets.

The diarrhoea was not so severe in either group that would give cause for concern from an economic aspect. No medium severe diarrhoea was observed.

In the piglet nursing phase the number of one-off, mild occurrences of diarrhoea in the zinc oxide group was 6, the number of medium severe occurrences was 9, while in the group receiving the preparation according to the invention the number of occurrences of mild diarrhoea was 8, and the number of medium severe occurrences was 15. The symptoms, therefore, affected 15% and 23% of the control group and 16% and 30% of the piglets treated with the feed according to the invention. The difference is not significant. Therefore, almost the same weaning diarrhoea prevention was achieved with the humic acid product supplemented with microelements than with the environmentally damaging zinc oxide.

With respect to the piglet performance achieved with the usually used zinc oxide medication (Zintestin Forte) and the feed supplement according to the invention and the animal health condition, the product according to the invention is at least as effective as the zinc oxide medication, however, the amount of zinc used and that finally pollutes the environment is a small fraction of the pollution created from the use of the zinc oxide medication according to the state of the art. There was no significant difference in the frequency of occurrence of piglet diarrhoea after weaning in average large-scale production conditions between the effects of the zinc oxide medication (Zintestin Forte) and the product according to the invention. The zinc concentration in the product according to the invention is 130 ppm, which is a small fraction of the 2600 ppm dose of the control substance zinc oxide (Zintestin Forte) recommended by the manufacturer and used in current practices.

The indicator determining the result of the post-weaning phase is the weight and quality of the piglets. During the post-weaning phase the piglets receiving the feed supplement according to the invention achieved the greatest daily weight gain as compared to the zinc oxide group with consideration to the fact that the animal health condition of the two groups was the same.

Therefore, the advantage of the invention as compared to the state of the art may be summarised as follows:
- The preparation according to the invention replaces the zinc oxide medication according to the state of the art in such a way that it reduces the amount of zinc polluting the environment to a small fraction and it may be effectively used to prevent and provide supplementary treatment for piglet diarrhoea.
- Beside a similar clinical effect to zinc oxide treatment, its use in the piglet raising phase results in greater weight increase than zinc oxide treatment.
- Its treatment costs are low, because the starting materials of the preparation may be obtained in large quantities at low prices, as compared to antibiotics, for example.
- The production of the preparation does not require complex equipment or chemical industry processes.

### List of figures:

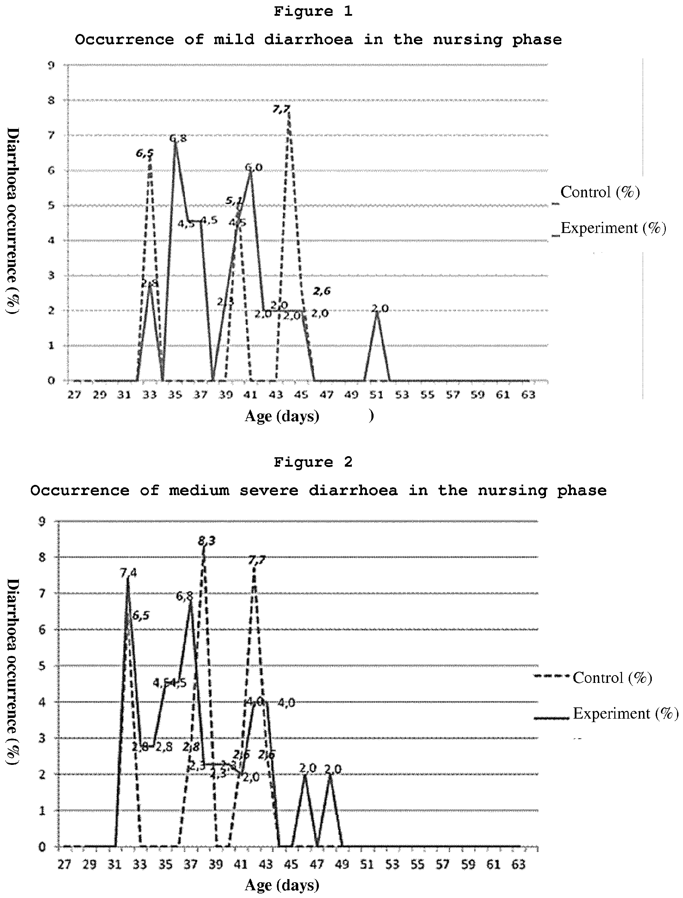

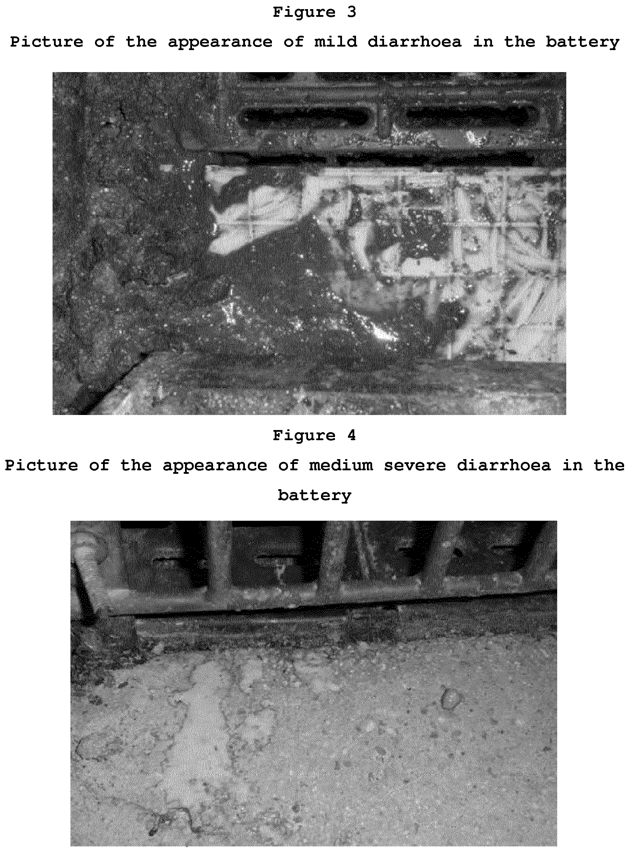

Drawing 1 /Figure 1 The graphic depiction of the occurrence of mild diarrhoea during the large-scale production experiment (example 3) during the nursing phase in the control and experiment groups in per cent as a function of time.
Drawing 1/Figure 2 The graphic depiction of the occurrence of medium severe diarrhoea during the large-scale production experiment (example 3) during the nursing phase in the control and experiment groups in per cent as a function of time.
Drawing 2/Figure 3 Picture of the appearance of mild diarrhoea in the battery (Photograph by Dr Jennifer Nagy)
Drawing 2/Figure 4 Picture of the appearance of medium severe diarrhoea in the battery (Photograph by Dr Jennifer Nagy) Drawing 3/Figure 5 Medium severe diarrhoea, the liquid faeces can be seen around the rectum. (Photograph by Dr Jennifer Nagy) Drawing 3/Figure 6 Picture of severe diarrhoea, contamination is prominent on the animal. The piglet is weak, in low spirits with a body posture suggesting abdominal pain. (Source: http://en.engormix.com/MA-pig-industry/health/articles/swine-dysentery-too-costly-t2437/165-p0.htm)

The invention is summarised in the following examples without restricting the invention to the following examples:

### Example 1

### Feed supplement preparation containing humic acids, zinc and copper

### Composition:

| Component: | |
|---|---|
| Peat (with min. 40% humic acid content) | 180 kg |
| Fulvic acid powder with 70% fulvic acid content | 90 kg |
| Crystalline Zn-sulphate monohydrate | 10-12 kg |
| Copper sulphate monohydrate | 17-19 kg |

The measured raw materials are placed into a mixing device. The device is started and the powder mixture is homogenised. The powder mixture obtained is packaged.

### Example 2

### Pilot-plant scale preliminary experiment for the prevention of artificially induced Escherichia coli diarrhoea

In the pilot plant experiment the clinical efficacy of the feed supplement preparation according to example 1 was examined in the prevention of artificially induced Escherichia coli diarrhoea in weaned piglets. The results obtained were compared with the zinc oxide medication method used in practice. The piglets taking part in the experiment received the aforementioned additives mixed in their feed for four weeks. On the 5^{th} day of feeding the piglets were infected on one occasion with a mixture of highly pathogenic E. coli strains isolated from clinical cases. The clinical symptoms resulting from the infection, their severity, the weight gain of the animals and the feed intake of the groups were studied. At the end of the study, the differences between the various treatment groups were compared.

### The experiment conditions:

During the study the piglets in the experiment were kept in a separate enclosure, in a conventional way. The animals were kept in groups, with 10 animals/group.

Lighting was maintained for 14 hours, which was partially natural and partially artificial (neon), the temperature was 15-25 °C, the relative humidity was (RAH) between 30-65%.

### The animal feed and water:

Feed and drinking water was provided ad *libitum* during the entire duration of the experiment. The quality of the feed was commercial quality coarse prestarter feed. The name and composition of the feed may be seen in table 5.

**Table 5**

| Name: |
|---|
| BabyProTM 28 prestarter feed mix |

| Guaranteed content: |
|---|
| Dry material % 88.00 |
| Raw protein % 18.00 |
| Raw fat (min) % 5.60 |
| Raw fibre (max) % 3.20 |
| Lysine % 1.45 |
| Methionine % 0.45 |
| Methionine+Cysteine % 0.84 |
| MEs MJ/kg 14.80 |
| DEs MJ/kg 15.10 |
| Ca % 0.64 |
| P % 0.61 |
| Na % 0.20 |
| Vitamin A NE/kg 10000 |
| Vitamin D NE/kg 1600 |
| Vitamin E mg/kg 110 |

The piglets in the experiment groups were fed with the different preparations for 4 weeks (see following point, experiment groups) in such a way that the preparations were mixed in advance in the feed. Feed intake was measured during the experiment per group and the average intake for the individual was calculated (see results) .

### EXPERIMENT GROUPS:

During the term of the experiment 5 groups of 10 piglets each were established according to treatment and infection (table 6). The grouping of the individuals was made on the basis of bodyweight, in such a way that when the experiment was started the average weight of the groups was nearly the same.

**Table 6**

| **Treatment** | **Infection** | **Group name** | **Group number** |
|---|---|---|---|
| none | none | Control | Group 1 |
| none | infected | Infected control | Group 2 |
| 1% humic acid product mixed into the feed for 4 weeks | infected | Humic | Group 3 |
| 2800 ppm zinc ion mixed into the feed for 4 weeks | infected | Zinc oxide | Group 4 |
| The use of the feed supplement according to the invention (1% humic acid product + 130 ppm zinc-ion + 150 ppm copper) mixed into the feed for 4 weeks | infected | Humic-zinc-copper | Group 5 |

During the experiment the piglets were artificially, orally infected on one occasion with an expressly swine-specific mixture of K88 antigen type *E. coli* strains.

Haemolysing *E. coli* bacterium isolates were used for the infection obtained from the strain collections of the Veterinary Diagnostics Institutes and the Epidemiology and Microbiology Department of the Veterinary Sciences Faculty of the Szent István University. These were previously isolated and collected from E. coli-caused diarrhoea in weaning age piglets. The strains were grouped into 10 groups according to epidemiological aspects, then during preliminary tests the five most pathogenic strains (numbered) were selected:
The epidemiological grouping of the isolates into 10 groups:
I./
   1./ 217/09
   2./ 218/09
   3./ 219/09
II./ 4./ 289/10 - (4)
III./
   5./ 292/10
   6./ 293/10
   7./ 294/10
IV.
   8./ 156/11
   9./ 157/11
V./ 10./ 192/11 - (5)
VI./ 11./ 211/11
VII./ 12./ 276/11
VIII./ K88 (F4) control 263: O8:K87:K88ab:H19 - (1)
IX./ F18 control 2173: O147: K+:H-, F18ac - (2)
X./ 987P (F6) control : 987P: O9:K103, :NM - (3)

A mixture of the numbered strains was used for the infection. The inoculum required for the infection experiments was prepared in the form of an Iso-Sensitest broth culture. The cultures were incubated for 5 hours at 37 °C in an incubator shaker, then the live bacteria count was determined using the broth dilution method. The live bacteria count of the inoculums in all cases was set to 5 x 10⁸ CFU/ml, then the bacteria suspension was transported to the infection site in sterile, 20-ml screw-cap test tubes cooled to 4 °C.

All the individuals of groups 2-5 received 2 ml of the above suspension using a probe.

### CLINICAL STUDIES:

### 1. Clinical study and observation

On arrival each individual was separately examined. Only clinically healthy animals were included in the experiment. The groups were monitored every day during the entire duration of the study and the general clinical condition of the individuals was examined. The piglets for signs of diarrhoea. The degree of diarrhoea was determined and given a score on the basis of the symptoms. A 0 score meant no diarrhoea, 1 mild diarrhoea, 2 medium severe and 3 severe diarrhoea. The results obtained in this way were recorded on a datasheet and evaluated (see below).

### 2. Bodyweight measurement

The bodyweight of the piglets was measured when they arrived and then twice a week using calibrated scales and weights. When the experiment was started the animals were grouped according to initial bodyweight. The measurement results were recorded on individual clinical datasheets and are presented in the Results section.

### 3. Feed intake

The intake of the feed provided ad libitum was determined daily, per group. The measurement results were recorded on the datasheets. The data gained during the experiment - feed intake, bodyweight change, clinical symptoms and severity - were collated and assessed using statistics tests.

The average weekly feed intake (kg) and standard deviation value:

**Table 7**

| **4** | **3** | **2** | **1** | **Group/We ek** |
|---|---|---|---|---|
| 3.771±0.39 | 3.714±0.38 | 2.286±0.38 | 1.486±0.157 | **1** |
| 3.429±0.39 | 3.714±0.38 | 2.286±0.38 | 1.486±0.157 | **2** |
| 4.686±0.552 | 4±0.462 | 2.4±0.4 | 1.486±0.157 | **3** |
| 3.771±0.605 | 3.257±0.538 | 2.4±0.4 | 1.6±0.383 | **4** |
| 3.543±0.428 | 3.371±0.39 | 2.457±0.276 | 1.6±0.383 | **5** |

From the third week the greatest feed intake was observed in the humic group (group 3). During the entire period of the study the group treated with the humic acid feed supplement supplemented with microelements according to example 1 (group 5) proved to have the lowest standard deviation value, which indicates that this group was the most homogenous.

The greatest standard deviation was found in the zinc oxide group (group 4), which may be due to the unreliable absorption of the zinc oxide. This partial result supported the hypothesis that the yield-increasing effect is not exerted directly by the zinc oxide but by influencing the microbiota. This was confirmed by the weight changes in the piglets after infection, where it was found that following artificial infection the piglets consuming feed medicated with zinc oxide (group 4) also lost weight, almost to the same extent as the group receiving the humic acid supplement (group 3). At the same time, due to the effect of the humic acid treatment supplemented with microelements according to the invention (group 5), during the week following infection the loss of weight in the piglets was just 50% of weight loss in the zinc dioxide group (group 4), and at the end of the experiment the weight of the piglets receiving humic acid treatment supplemented with microelements according to the invention (group 5) was the same as the weight of the piglets receiving zinc oxide treatment. In other words, with respect to the so-called yield-increasing effect, the humic acid product supplemented with microelements according to the invention was equivalent to the currently used zinc oxide.

The following tables present the individual bodyweight data (kg) of the experiment piglets and the average values at the various measurement times.

**Table 8 group 1 (control)**

| **17 Nov** | **14 Nov** | **10 Nov** | **07 Nov** | **03 Nov** | **31 Oct** | **27 Oct** | **24 Oct** | **20 Oct** | **Animal No** |
|---|---|---|---|---|---|---|---|---|---|
| 7.5 | 9 | 8 | 7.5 | 7.5 | 7 | 4.5 | 4 | 6 | 1 |
| 7 | 8 | 7.5 | 7 | 7 | 7 | 5.5 | 5.5 | 6.5 | 2 |
| 7.5 | 9 | 8 | 7 | 7 | 6 | 4.5 | 5.5 | 5.8 | 3 |
| 7 | 8 | 8 | 8 | 8 | 9.5 | 6.5 | 6.5 | 7.3 | 4 |
| 10 | 10 | 8 | 7 | 7.5 | 8 | 5.5 | 5 | 6 | 5 |
| 5 | 6 | 6.5 | 6 | 7 | 6 | 3.5 | 3 | 4 | 6 |
| 10 | 11.5 | 9 | 8.5 | 8 | 9 | 6 | 6 | 6.2 | 7 |
| 7.5 | 10 | 8.5 | 8.5 | 8 | 9 | 6 | 7 | 6.7 | 8 |
| 5.5 | 7 | 7 | 6 | 6 | 6 | 4 | 5 | 5.3 | 9 |
| 5 | 6 | 5.5 | 6 | 5 | 5.5 | 3.5 | 4 | 4 | 10 |
| **7.20** | **8.45** | **7.60** | **7.15** | **7.10** | **7.30** | **4.95** | **5.15** | **5.78** | **Average** |
| **1.78** | **1.80** | **1.02** | **0.97** | **0.97** | **1.48** | **1.09** | **1.23** | **1.08** | **Dist.** |

**Table 9 group 2 (infected control)**

| **17 Nov** | **14 Nov** | **10 Nov** | **07 Nov** | **03 Nov** | **31 Oct** | **27 Oct** | **24 Oct** | **20 Oct** | **Animal No** |
|---|---|---|---|---|---|---|---|---|---|
| | | | | | | | 6.5 | 6.2 | 1* |
| 10.5 | 12 | 10.5 | 10 | 9 | 8 | 7 | 7 | 8 | 2 |
| | | | | | | | 6 | 5.1 | 3* |
| 8 | 10 | 8.5 | 7 | 8.5 | 8 | 5 | 5 | 5.9 | 4 |
| 6 | 6.5 | 6 | 5 | 7 | 6 | 4 | 3 | 4 | 5 |
| 7 | 8 | 7.5 | 7 | 8.5 | 8 | 6 | 4 | 5.5 | 6 |
| 9 | 11 | 9 | 7.5 | 9 | 8 | 6 | 5 | 6.4 | 7 |
| 8 | 9.5 | 8.5 | 7.5 | 9 | 8 | 6 | 5 | 5.5 | 8 |
| 7 | 8.5 | 7.5 | 6 | 7 | 6 | 5 | 5 | 5.5 | 9 |
| 8 | 9 | 7.5 | 6.5 | 7.5 | 7 | 5.5 | 4.5 | 6 | 10 |
| **7.94** | **9.31** | **8.13** | **7.06** | **8.19** | **7.38** | **5.56** | **5.10** | **5.81** | **Average** |
| **1.37** | **1.73** | **1.33** | **1.45** | **0.88** | **0.92** | **0.90** | **1.17** | **1.02** | **Dist.** |

**Table 10 group 3 (humic)**

| **17 Nov** | **14 Nov** | **10 Nov** | **07 Nov** | **03 Nov** | **31 Oct** | **27 Oct** | **24 Oct** | **20 Oct** | **Animal No** |
|---|---|---|---|---|---|---|---|---|---|
| 9 | 9.5 | 9 | 8 | 9 | 8 | 5.5 | 4 | 6.7 | 1 |
| 7.5 | 7 | 7 | 6 | 6 | 6 | 4.5 | 4 | 5.7 | 2 |
| 7 | 7.5 | 7 | 6.5 | 8 | 7 | 4 | 4 | 4.5 | 3 |
| 9 | 8 | 8 | 7 | 8.5 | 8 | 5 | 6 | 6.6 | 4 |
| 8 | 8.5 | 8.5 | 8 | 9 | 8 | 5 | 6 | 6.5 | 5 |
| 8.5 | 8 | 8 | 6.5 | 8 | 7 | 5 | 4.5 | 6 | 6 |
| 8.5 | 8 | 7 | 6 | 7.5 | 7 | 4.5 | 4.5 | 6 | 7 |
| 8 | 7.5 | 7 | 6 | 7 | 7 | 5 | 4.5 | 6 | 8 |
| 7 | 7.5 | 7 | 6 | 7.5 | 7 | 4 | 5.5 | 6 | 9 |
| 9.5 | 10 | 9 | 8.5 | 9 | 8 | 4.5 | 5 | 5 | 10 |
| **8.20** | **8.15** | **7.75** | **6.85** | **7.95** | **7.30** | **4.70** | **4.80** | **5.90** | **Average** |
| **0.86** | **0.94** | **0.86** | **0.97** | **0.98** | **0.67** | **0.48** | **0.79** | **0.69** | **Dist.** |

**Table 11 group 4 (zinc oxide)**

| **17 Nov** | **14 Nov** | **10 Nov** | **07 Nov** | **03 Nov** | **31 Oct** | **27 Oct** | **24 Oct** | **20 Oct** | **Animal No** |
|---|---|---|---|---|---|---|---|---|---|
| 10.5 | 11 | 10 | 11.5 | 11.3 | 10 | 8.5 | 8 | 7 | 1 |
| 4.5 | 5 | 4.5 | 4.5 | 6 | 5 | 5 | 5.5 | 4.5 | 2 |
| 11.5 | 12 | 10 | 10 | 11 | 9 | 6 | 6 | 6 | 3 |
| | | | | | | | 6.5 | 7.5 | 4* |
| 8.5 | 9.5 | 8 | 7 | 9 | 8 | 6.5 | 6 | 7.5 | 5 |
| 9 | 9.5 | 9 | 8 | 9 | 7 | 6 | 6 | 7 | 6 |
| 9 | 10 | 9 | 8 | 9 | 7 | 6.5 | 7 | 4 | 7 |
| 7 | 8 | 7 | 6 | 7 | 6 | 4.5 | 5 | 5 | 8 |
| | | | | | | | 4.5 | 5 | 9* |
| 10 | 10 | 9 | 9 | 9.5 | 9 | 8 | 7 | 6.5 | 10 |
| **8.75** | **9.38** | **8.31** | **8.00** | **8.98** | **7.63** | **6.38** | **6.15** | **6.00** | **Average** |
| **2.19** | **2.12** | **1.83** | **2.22** | **1.79** | **1.69** | **1.36** | **1.03** | **1.29** | **Dist.** |

**Table 12 group 5 (humic-zinc-copper)**

| **17 Nov** | **14 Nov** | **10 Nov** | **07 Nov** | **03 Nov** | **31 Oct** | **27 Oct** | **24 Oct** | **20 Oct** | **Animal No** |
|---|---|---|---|---|---|---|---|---|---|
| 11 | 11.5 | 9 | 8 | 7 | 8 | 7.5 | 6.5 | 7.5 | 1 |
| | | | | | | | 4 | 4.5 | 2* |
| 6.5 | | 6.5 | 6 | 7 | 6 | 5 | 4 | 5 | 3 |
| 11.5 | 12 | 10.5 | 11.5 | 13 | 10 | 8 | 8.5 | 7.5 | 4 |
| 5 | 4.5 | 5.5 | 5 | 6 | 5 | 3.5 | 3.5 | 3 | 5 |
| | | | | | | | 4 | 5.5 | 6* |
| 13 | 12 | 11.5 | 11.5 | 11.5 | 10 | 8 | 7 | 17.5 | 7 |
| 7.5 | 9 | 8 | 8 | 8.5 | 8 | 6 | 6 | 6 | 8 |
| 7 | 8 | 7 | 6 | 7 | 6 | 4.5 | 4 | 4.5 | 9 |
| 10.5 | 11.5 | 9.5 | 10 | 9.5 | 8 | 7 | 7 | 7 | 10 |
| **9.00** | **9.44** | **8.44** | **8.25** | **8.69** | **7.63** | **6.19** | **5.45** | **5.80** | **Average** |
| **2.85** | **2.78** | **2.06** | **2.54** | **2.48** | **1.85** | **1.71** | **1.76** | **1.57** | **Dist.** |

### Clinical symptoms

Every piglet was clinically examined on the first day of the experiment. The animals were well developed and lively, and were in average-good condition. General condition and appetite were also good. No animal exhibited any symptoms of disease.

Up until the 5^{th} day (infection) of the feeding experiment every animal exhibited a clinical picture according to that described above during the daily examinations.

Within 2-4 hours of the infections taking place 6 artificially infected individuals died in a peracute way. Initially the clinical symptoms were difficult, wheezing respiration, unsteady gait, then these were followed by tonic clonic spasms. A frothy, reddish secretion appeared in the mouth cavity and in the nose, after this the affected individuals quickly died. On the basis of the anamnesis data, the fast progression and the clinical symptoms, endotoxin shock was determined as the cause of death.

The animals in the infected groups had varying degrees (severity) of diarrhoea depending on the feed additive given. The most severe symptoms were observed in the infected piglets that did not receive treatment (group 2, infected control), while during this period the piglets in the control group (group 1) were without any symptoms all the way through the experiment. (It should be noted that in week 4 of the feeding experiment a number of individuals in the control group had mild diarrhoea.)

In the case of the groups receiving the various treatments, the number of seriously ill piglets was less on average, and the symptoms (listlessness, lack of appetite) were found to be milder as compared to the infected control group. The piglets in the humic group (group 3) had more severe symptoms overall and the duration of the diarrhoea was longer, while the individuals in the zinc oxide group (group 4) only had mild symptoms, and the symptoms disappeared sooner. The values of the piglets treated with the preparation according to the invention (group 5) fell between those of the previous two groups. The following tables summarise the number of individuals with diarrhoea and its severity during the two weeks following infection.

**Table 13 group 1 (control)**

| **14** | **13** | **12** | **11** | **10** | **9** | **8** | **7** | **6** | **5** | **4** | **3** | **2** | **1** | **Animal No/day** |
|---|---|---|---|---|---|---|---|---|---|---|---|---|---|---|
| 0 | 0 | 0 | 0 | 0 | 0 | 0 | 0 | 0 | 0 | 0 | 0 | 0 | 0 | **1** |
| 0 | 0 | 0 | 0 | 0 | 0 | 0 | 0 | 0 | 0 | 0 | 0 | 0 | 0 | **2** |
| 0 | 0 | 0 | 1 | 0 | 0 | 0 | 0 | 0 | 0 | 0 | 0 | 0 | 0 | **3** |
| 0 | 0 | 0 | 0 | 0 | 0 | 0 | 0 | 0 | 0 | 0 | 0 | 0 | 0 | **4** |
| 1 | 2 | 0 | 0 | 0 | 0 | 0 | 0 | 0 | 0 | 0 | 0 | 0 | 0 | **5** |
| 1 | 1 | 2 | 1 | 1 | 0 | 0 | 0 | 0 | 0 | 0 | 0 | 0 | 0 | **6** |
| 0 | 0 | 0 | 0 | 0 | 0 | 0 | 0 | 0 | 0 | 0 | 0 | 0 | 0 | **7** |
| 0 | 0 | 0 | 0 | 0 | 0 | 0 | 0 | 0 | 0 | 0 | 0 | 0 | 0 | **8** |
| 1 | 2 | 1 | 0 | 0 | 0 | 0 | 0 | 0 | 0 | 0 | 0 | 0 | 0 | **9** |
| 1 | 1 | 2 | 1 | 1 | 0 | 0 | 0 | 0 | 0 | 0 | 0 | 0 | 0 | **10** |

| | | | | | | | | | | | | | | |
|---|---|---|---|---|---|---|---|---|---|---|---|---|---|---|
| (0=no diarrhoea, 1=mild, 2=medium severe, 3=severe diarrhoea) | | | | | | | | | | | | | | |

**Table 14 group 2 (infected control)**

| **14** | **13** | **12** | **11** | **10** | **9** | **8** | **7** | **6** | **5** | **4** | **3** | **2** | **1** | **Animal No/day** |
|---|---|---|---|---|---|---|---|---|---|---|---|---|---|---|
| | | | | | | | | | | | | | | **1*** |
| 0 | 0 | 1 | 0 | 0 | 1 | 1 | 0 | 2 | 3 | 2 | 0 | 2 | 0 | **2** |
| | | | | | | | | | | | | | | **3*** |
| 1 | 1 | 1 | 2 | 1 | 2 | 3 | 2 | 3 | 3 | 2 | 0 | 3 | 0 | **4** |
| 1 | 1 | 1 | 3 | 1 | 2 | 3 | 2 | 2 | 3 | 2 | 3 | 2 | 0 | **5** |
| 1 | 1 | 2 | 3 | 3 | 2 | 1 | 2 | 3 | 3 | 2 | 1 | 1 | 0 | **6** |
| 1 | 1 | 1 | 0 | 0 | 1 | 2 | 2 | 1 | 2 | 3 | 2 | 2 | 0 | **7** |
| 2 | 3 | 2 | 0 | 0 | 1 | 2 | 2 | 2 | 3 | 3 | 0 | 3 | 0 | **8** |
| 1 | 2 | 2 | 1 | 2 | 2 | 1 | 1 | 2 | 2 | 3 | 3 | 2 | 0 | **9** |
| 1 | 1 | 2 | 2 | 1 | 1 | 2 | 2 | 1 | 2 | 2 | 0 | 3 | 0 | **10** |

| | | | | | | | | | | | | | | |
|---|---|---|---|---|---|---|---|---|---|---|---|---|---|---|
| * Died of endotoxin shock as a result of the infection. (0=no diarrhoea, 1=mild, 2=medium severe, 3=severe diarrhoea) | | | | | | | | | | | | | | |

**Table 15 group 3 (humic)**

| **14** | **13** | **12** | **11** | **10** | **9** | **8** | **7** | **6** | **5** | **4** | **3** | **2** | **1** | **Animal No/day** |
|---|---|---|---|---|---|---|---|---|---|---|---|---|---|---|
| 0 | 1 | 1 | 0 | 0 | 1 | 2 | 1 | 0 | 1 | 2 | 2 | 0 | 0 | **1** |
| 0 | 1 | 1 | 1 | 1 | 2 | 1 | 2 | 2 | 0 | 1 | 3 | 1 | 0 | **2** |
| 0 | 1 | 1 | 0 | 0 | 0 | 0 | 0 | 1 | 1 | 2 | 2 | 2 | 0 | **3** |
| 0 | 1 | 1 | 0 | 0 | 0 | 0 | 0 | 1 | 1 | 1 | 2 | 2 | 0 | **4** |
| 0 | 1 | 2 | 0 | 0 | 0 | 0 | 0 | 1 | 1 | 2 | 2 | 0 | 0 | **5** |
| 0 | 1 | 2 | 0 | 0 | 0 | 0 | 0 | 0 | 0 | 1 | 2 | 2 | 0 | **6** |
| 0 | 1 | 1 | 0 | 0 | 1 | 1 | 2 | 1 | 2 | 1 | 2 | 1 | 0 | **7** |
| 0 | 1 | 1 | 1 | 1 | 2 | 0 | 1 | 1 | 0 | 0 | 0 | 2 | 0 | **8** |
| 0 | 1 | 2 | 2 | 1 | 2 | 2 | 1 | 2 | 2 | 1 | 2 | 1 | 0 | **9** |
| 0 | 1 | 1 | 0 | 0 | 0 | 1 | 2 | 1 | 2 | 1 | 1 | 2 | 0 | **10** |

| | | | | | | | | | | | | | | |
|---|---|---|---|---|---|---|---|---|---|---|---|---|---|---|
| (0=no diarrhoea, 1=mild, 2=medium severe, 3=severe diarrhoea) | | | | | | | | | | | | | | |

**Table 16 group 4 (zinc oxide)**

| **14** | **13** | **12** | **11** | **10** | **9** | **8** | **7** | **6** | **5** | **4** | **3** | **2** | **1** | **Animal No/day** |
|---|---|---|---|---|---|---|---|---|---|---|---|---|---|---|
| 0 | 0 | 0 | 0 | 0 | 0 | 0 | 0 | 0 | 0 | 1 | 1 | 1 | 0 | **1** |
| 0 | 0 | 0 | 0 | 0 | 0 | 0 | 0 | 1 | 1 | 1 | 2 | 1 | 0 | **2** |
| 0 | 0 | 0 | 0 | 0 | 0 | 0 | 0 | 0 | 0 | 0 | 0 | 0 | 0 | **3** |
| | | | | | | | | | | | | | | **4*** |
| 0 | 0 | 0 | 0 | 0 | 0 | 0 | 0 | 0 | 0 | 0 | 0 | 0 | 0 | **5** |
| 0 | 0 | 0 | 0 | 0 | 0 | 0 | 0 | 0 | 0 | 0 | 0 | 0 | 0 | **6** |
| 0 | 0 | 0 | 0 | 0 | 0 | 0 | 0 | 0 | 0 | 1 | 1 | 0 | 0 | **7** |
| 0 | 0 | 0 | 0 | 0 | 0 | 0 | 0 | 0 | 1 | 1 | 2 | 1 | 0 | **8** |
| | | | | | | | | | | | | | | **9*** |
| 0 | 0 | 0 | 0 | 0 | 0 | 0 | 0 | 0 | 0 | 0 | 0 | 1 | 0 | **10** |

| | | | | | | | | | | | | | | |
|---|---|---|---|---|---|---|---|---|---|---|---|---|---|---|
| * Died of endotoxin shock as a result of the infection. (0=no diarrhoea, 1=mild, 2=medium severe, 3=severe diarrhoea) | | | | | | | | | | | | | | |

**Table 17 group 5 (humic-zinc-copper)**

| **14** | **13** | **12** | **11** | **10** | **9** | **8** | **7** | **6** | **5** | **4** | **3** | **2** | **1** | **Animal No/day** |
|---|---|---|---|---|---|---|---|---|---|---|---|---|---|---|
| 0 | 0 | 0 | 0 | 0 | 0 | 0 | 1 | 1 | 1 | 2 | 1 | 1 | 0 | **1** |
| | | | | | | | | | | | | | | **2*** |
| 0 | 0 | 0 | 0 | 0 | 0 | 0 | 0 | 0 | 1 | 2 | 1 | 2 | 0 | **3** |
| 0 | 0 | 0 | 0 | 0 | 0 | 0 | 0 | 0 | 0 | 1 | 2 | 1 | 0 | **4** |
| 0 | 0 | 1 | 0 | 0 | 0 | 0 | 0 | 0 | 0 | 0 | 1 | 1 | 0 | **5** |
| | | | | | | | | | | | | | | **6*** |
| 0 | 0 | 0 | 0 | 0 | 0 | 0 | 0 | 0 | 0 | 0 | 0 | 0 | 0 | **7** |
| 0 | 0 | 0 | 0 | 0 | 0 | 0 | 0 | 0 | 0 | 0 | 0 | 1 | 0 | **8** |
| 0 | 0 | 1 | 1 | 1 | 0 | 1 | 1 | 0 | 1 | 1 | 2 | 1 | 0 | **9** |
| 0 | 0 | 0 | 0 | 0 | 0 | 0 | 0 | 0 | 0 | 0 | 0 | 0 | 0 | **10** |

| | | | | | | | | | | | | | | |
|---|---|---|---|---|---|---|---|---|---|---|---|---|---|---|
| * Died of endotoxin shock as a result of the infection. (0=no diarrhoea, 1=mild, 2=medium severe, 3=severe diarrhoea) | | | | | | | | | | | | | | |

Therefore, from the point of view of clinical efficacy, with regard to the number of diarrhoea days, the humic acid product supplemented with microelements according to the invention (according to example 1) was hardly 10% less effective than the currently used zinc oxide.

With respect to the severity of diarrhoea, the effect of the humic acid feed supplement with microelements according to the invention was just 4% weaker than zinc oxide when comparing the results to the infected control group. However, the severity of the diarrhoea is a subjective indicator, judging it is not uniform among livestock breeders and vets.

This is shown by that at the end of the experiment the greatest weight gain was in the group receiving humic acid supplemented with microelements according to the invention (group 5). This is deemed important because during the nursery period it is not the severity of the diarrhoea that is decisive but the weight of the piglets when they go to the post-weaning phase. Production results show that - 250 g bodyweight difference in the case of 8-week-old piglets - the use of the feed supplement containing humic acid, zinc and copper is preferable to zinc oxide. In spite of the diarrhoea symptoms lasting two days more, 8-week-old piglets were produced that had a greater weight due to the effect of the humic acid and microelement treatment according to the invention.

### Example 4

### Large-scale experiment for the use of a feed supplement containing humic acids, zinc and copper compounds

The large-scale experiment was carried out at the Herceghalom Testing Station of the National Agricultural Research and Innovation Centre, Research Institute for Animal Breeding, Nutrition and Meat Science. The experiment involved 74 experiment and 53 control piglets of mixed sex aged from 10 days to 55 days, which were from a Hungarian Large White x Hungarian Landrace population.

The experiment piglets were individually measured during suckling per litter at the ages of 10 and 28 days. The amount of feed intake during this period was measured per litter.

Weaning took place at an average of 32 days. The piglets were placed in post-weaning batteries at equal sex ratio. When the piglets were moved care was taken to ensure that a maximum of 5 piglets from other sows were placed in the same pen, which was carried out to model actual large-scale production conditions. 10 repetitions were performed for the experiment group and 9 repetitions for the control group. The piglets were individually weighed on the day of weaning (32 days old) and after 23 days of experiment feeding (55 days old).

Experiment feeds: The piglets were fed starter piglet feed from 10 days of age until the completion of the experiment. The control group of piglets was fed with the starter piglet feed used at the farm, supplemented with Zintestin Forte premix containing zinc oxide. The piglets in the experiment group received the same feed, which, instead of the Zintestin Forte premix containing zinc oxide, was supplemented with the humic acid additive with microelements according to the invention. The animals could feed ad libitum from a self-feeder and drink from bite ball valves.

### Groups

**Group 1:** Group treated with zinc oxide medication (Zintestin Forte premix A.U.V., Industrial Veterinaria S.A., Spain).
**Group 2:** The group treated with the product according to example 1

Data recorded: the following production parameters were recorded at the following times

### Suckling phase

10-day piglet weight
10-day initial population number
28-day piglet weight
28-day population number
feed intake between days 10-28
deaths
health condition
diarrhoea frequency, treatment frequency
Piglet raising phase
Piglet weaning weight
Weaning piglet population number
55-day piglet weight
55-day piglet population number
feed intake between days 32-55
deaths
heath condition
diarrhoea frequency, treatment frequency
Biometric calculations: The biometric calculations (basic statistic, variance analysis, T-test) were performed using the SPSS program.

**Table 18 The composition of the starter piglet feed used and the calculated nutrient content (%)**

| **Group 2 Experiment** | **Group 1 Control** | **Name** |
|---|---|---|
| 50.0 | 50.0 | **Corn** |
| 9.0 | 9.0 | **Wheat** |
| 10.0 | 10.0 | **Barley** |
| 20.0 | 20.0 | **Extr. Soya grits** |
| 10.0 | 10.0 | **Sano Suggi Owa** |
| | 1.0 | **Zintestin Forte premix A.U.V.** |
| 1.0 | - | **Humic acid preparation (Alpha-Vet)** |
| | | **Calculated nutrient content** |
| 88.0 | 88.0 | **Dry material** |
| 13.9 | 13.9 | **DEs, MJ/kg** |
| 17.0 | 17.0 | **Raw protein** |
| 4.5 | 4.5 | **Raw fat** |
| 2.7 | 2.7 | **Raw fibre** |
| 5.6 | 5.6 | **Ash** |
| 0.18 | 0.18 | **Na** |
| 0.84 | 0.84 | **Ca** |
| 0.54 | 0.54 | **P** |
| 1.17 | 1.17 | **Lysine** |
| 0.42 | 0.42 | **Methionine** |
| 20000 | 20000 | **Vitamin A, NE/kg** |
| 2000 | 2000 | **Vitamin D3, NE/kg** |
| 150 | 150 | **Vitamin E (alpha tocopherol), mg/kg** |
| 250 | 2730 | **+Zn (ZnO), mg/kg** |

The result of the chemical analysis of the experiment feeds:
The following table illustrates the result of the Weende analysis of the feed mix.

**Table 19 Chemical composition of the experiment feed mixes, %**

| **Starter piglet feed** | | |
|---|---|---|
| Group 2 **Experiment** | Group 1 **Control** | |
| 88.65 | 89.03 | **Dry material** |
| 17.32 | 17.15 | **Raw protein** |
| 4.00 | 4.14 | **Raw fat** |
| 2.65 | 2.41 | **Raw fibre** |
| 5.63 | 5.28 | **Ash** |

It is shown from the data that the measured data are nearly identical to the data calculated in table 18. This is especially important in the case of raw protein, as in the case of a young animal the raw protein content of the feed is the most important nutrient from the point of view of protein synthesis. Therefore, we fed the piglets in accordance with their needs.

The performance indicators of the piglets in the suckling phase were as follows:

**Table 20**

| **Daily weight gain** | **Total weight gained** | **28-day weight** | **10-day weight** | |
|---|---|---|---|---|
| 195.1 g/day | 3.58 kg | 6.50 kg | 3.16 kg | **Group 1 (control)** |
| 182.0 g/day | 3.42 kg | 6.12 kg | 12,99 kg | **Group 2 (experiment)** |
| -13.1 g/day | -0.16 kg | -0.38 kg | -0.17 kg | **Difference compared to control** |

The average daily weight gain was 8% greater in the control group, the difference is not significant.

On examining the results it may be determined that during the suckling piglet phase the feed supplement according to the invention (according to example 1) did not provide the yield-increasing effect that the zinc oxide medication (Zintestin Forte) did. The suckling piglets eat the preparation according to the invention and do not reject it. The preparation according to the invention does not influence the intake of the prestarter piglet feed by the suckling piglets in the farrowing pen.

When moving the piglets after weaning the equal sex ratio was set for the two groups, and the runt piglets were taken out of the experiment. The control group piglets were placed in 9 pens and the treated piglets were placed in 10 pens. The piglet rearing experiment included 39 piglets in the control group and 50 piglets in the experiment group.

The performance indicators of the piglets in the post-weaning phase were as follows:

**Table 21**

| **Daily weight gain** | **Total weight gained** | **Closin g weight** | **Initial weight** | |
|---|---|---|---|---|
| 252.39 g/day | 6.06 kg | 14.06 kg | 8.00 kg | **Group 1 (control)** |
| 306.0 g/day | 7.34 kg | 15.12 kg | 7.78 kg | **Group 2 (experiment)** |
| +53.61 g/day | +1.28 kg | + 1.06 kg | -0.22 | **Difference compared to control** |

The duration of the experiment was 23 days, from weaning (average age of 32 days) until the piglets reached the average age of 55 days. The daily weight gain, as opposed to the period before weaning, was 12% greater in the experiment group.

In the post-weaning phase the preparation according to the invention had a clearly positive effect on the growing vigour of the weaned piglets. A greater yield-increasing effect was achieved with the supplement according to the invention than with the currently used zinc oxide. This verified the yield-increasing effect experienced in the pilot-scale experiment and confirmed that the humic acid feed supplement with microelements according to the invention is a yield-increasing product equivalent to or more favourable than zinc oxide.

### Piglet health condition:

### Deaths in the experiment

Deaths only occurred in the suckling phase, the causes of the deaths are summarised in table 22. 2 piglets died in the control group and 5 piglets died in the experiment group.

**Table 22 Suckling piglet deaths**

| **Cause of death** | **Deceased piglets (no)** | **Sow tag number** | |
|---|---|---|---|
| Pyelitis | 1 | 1579 | |
| Pneumonia | 1 | 1519 | |
| | **2** | | **Group 1 (Control) total** |
| Gastroenteritis, cardiac tamponade | 1 | 11565 | |
| Enteritis, pneumonia | 2 | 1498 | |
| Gastritis | 1 | 1545 | |
| Crushed | 1 | 1467 | |
| | **5** | | **Group 2 (Experimen t) total** |

### Occurrence of diarrhoea in the experiment:

The occurrence of diarrhoea was monitored in both phases of the experiment.

The diarrhoea was classed into three categories of severity:
I. Mild: the faeces are slightly more liquid than normal. The animal is in good condition, lively. No more than a maximum of 1/litter or pen, diarrhoea does not occur again that day.
II. Medium severe diarrhoea: liquid faeces with high moisture content. The animal appears well. Signs of fluid faeces can be seen around the rectum. No more than a maximum of 1/litter or pen, diarrhoea does not occur again that day.
III. Severe diarrhoea: large quantity of liquid faeces. Prominent contamination on the animal. The piglet is weak, in low spirits with a body posture suggesting abdominal pain or a (sudden) deterioration of condition can be seen.

All animals showing signs of diarrhoea during the experiment were treated with antibiotics (Betamox LA inj. A.U.V., Norbrook®, United Kingdom).

Table 23 shows the summary data relating to the occurrence of diarrhoea in the suckling phase.

**Table 23 The occurrence of diarrhoea in the suckling piglet phase**

| | Diarrhoea frequency | | Diarrhoea severity | |
|---|---|---|---|---|
| 2 | 1 | | | |
| 0 | 10 | piglets (no) | mild | Control n=53 |
| 0 | 18,9 | piglets (%) | | |
| 0 | 0 | piglets (no) | medium severe | |
| 0 | 0 | piglets (%) | | |
| 0 | 45 | piglets (no) | mild | experiment n=74 |
| 0 | 60,8 | piglets (%) | | |
| 0 | 0 | piglets (no) | medium severe | |
| 0 | 0 | piglets (%) | | |

During the suckling phase 19% of the control group and 61% of the animals treated with the preparation according to the invention had mild, non-repeating diarrhoea. It was observed that in the group treated with the preparation according to the invention diarrhoea mainly occurred in the suckling piglets in the farrowing pen after they reached 18 days of age, which may suggest that the composition of the sow's milk changes due to the reactivated ovaries and the reoccurring ovarian hormones, and that it is this that generates the diarrhoea in the more sensitive piglets.

Medium severe diarrhoea was not observed.
Table 24 shows the occurrence of mild diarrhoea with the progression of age of the piglets, the occurrence according to the days in the farrowing pen in detail.

**Table 24 The occurrence of mild diarrhoea in the farrowing pen**

| | | Experiment | | | | Control |
|---|---|---|---|---|---|---|
| % | Diarrhoea (no) | Piglet population | % | Diarrhoea (no) | Piglet population | Age |
| 0 | 0 | 74 | 0 | 0 | 54 | 10 |
| 0 | 0 | 74 | 0 | 0 | 53 | 11 |
| 0 | 0 | 74 | 0 | 0 | 53 | 12 |
| 0 | 0 | 72 | 0 | 0 | 53 | 13 |
| 0 | 0 | 72 | 18.9 | 10 | 53 | 14 |
| 0 | 0 | 72 | 0 | 0 | 53 | 15 |
| 0 | 0 | 71 | 0 | 0 | 53 | 16 |
| 0 | 0 | 71 | 0 | 0 | 53 | 17 |
| 0 | 0 | 71 | 0 | 0 | 53 | 18 |
| 14.1 | 10 | 71 | 0 | 0 | 53 | 19 |
| 0 | 0 | 71 | 0 | 0 | 53 | 20 |
| 0 | 0 | 71 | 0 | 0 | 53 | 21 |
| 15.5 | 11 | 71 | 0 | 0 | 53 | 22 |
| 0 | 0 | 71 | 0 | 0 | 53 | 23 |
| 0 | 0 | 71 | 0 | 0 | 52 | 24 |
| 0 | 0 | 71 | 0 | 0 | 52 | 25 |
| 18.3 | 13 | 71 | 0 | 0 | 51 | 26 |
| 15 | 11 | 71 | 0 | 0 | 41 | 27 |
| 0 | 0 | 71 | 0 | 0 | 41 | 28 |
| 0 | 0 | 59 | 0 | 0 | 41 | 29 |
| 0 | 0 | 28 | 0 | 0 | 34 | 30 |
| 0 | 0 | 28 | 0 | 0 | 21 | 31 |
| 0 | 0 | 28 | 0 | 0 | 10 | 32 |
| 0 | 0 | 17 | 0 | 0 | 10 | 33 |
| 0 | 0 | 16 | 0 | 0 | 4 | 34 |
| 0 | 0 | 6 | 0 | 0 | 4 | 35 |
| 0 | 0 | 6 | 0 | 0 | 4 | 36 |
| 0 | 0 | 6 | 0 | 0 | 4 | 37 |
| 0 | 0 | 6 | 0 | 0 | 4 | 38 |
| 0 | 0 | 6 | | | | 39 |
| 0 | 0 | 6 | | | | 40 |

The occurrence of diarrhoea in the post-weaning phase is presented in table 25.

The occurrence of mild diarrhoea in the post-weaning phase is illustrated in Figure 1 (drawing 1) and of medium severe diarrhoea in Figure 2 (drawing 1).

**Table 25 The occurrence of diarrhoea in the post-weaning phase**

| | | Diarrhoea frequency | | | |
|---|---|---|---|---|---|
| 3 | 2 | 1 | | Diarrhoea category | |
| 0 | 1 | 6 | piglet (no) | mild | Control n=39 |
| 0 | 2.6 | 15.4 | piglet (%) | | |
| 0 | 1 | 9 | piglet (no) | medium severe | |
| 0 | 2.6 | 23.1 | piglet (%) | | |
| 1 | 4 | 8 | piglet (no) | mild | Experiment n=50 |
| 2,0 | 8.0 | 16.0 | piglet (%) | | |
| 0 | 3 | 15 | piglet (no) | medium severe | |
| 0 | 6.0 | 30.0 | piglet (%) | | |

In the control group the number of one-off, mild occurrences of diarrhoea was 6, the number of medium severe occurrences was 9, while in the experiment group the number of occurrences of mild diarrhoea was 8, and the number of medium severe occurrences was 15. The symptoms, therefore, affected 15% and 23% of the control group and 16% and 30% of the treated piglets. The difference is not significant. Therefore, almost the same weaning diarrhoea prevention was achieved with the humic acid product supplemented with microelements than with the environmentally damaging zinc oxide.

The occurrence of mild and medium severe diarrhoea in the post-weaning phase is summarised in tables 26 and 27. The symptoms primarily occurred between the ages of 32-45 days.

**Table 26**

| The occurrence of mild diarrhoea in the post-weaning phase | | | | | | |
|---|---|---|---|---|---|---|
| | Control | | | Experiment | | |
| | Mild | | | Mild | | |
| Age | Piglet no | Diarrhoea (no) | Control % | Piglet no | Diarrhoea (no) | Experiment % |
| 27 | 8 | 0 | 0 | | | |
| 28 | 8 | 0 | 0 | | | |
| 29 | 8 | 0 | 0 | 7 | 0 | 0 |
| 30 | 17 | 0 | 0 | 26 | 0 | 0 |
| 31 | 26 | 0 | 0 | 27 | 0 | 0 |
| 32 | 31 | 0 | 0 | 27 | 0 | 0 |
| 33 | 31 | 2 | 6.5 | 36 | 1 | 2.8 |
| 34 | 36 | 0 | 0 | 36 | 0 | 0 |
| 35 | 36 | 0 | 0 | 44 | 3 | 6.8 |
| 36 | 36 | 0 | 0 | 44 | 2 | 4.5 |
| 37 | 36 | 0 | 0 | 44 | 2 | 4.5 |
| 38 | 36 | 0 | 0 | 44 | 0 | 0 |
| 39 | 39 | 0 | 0 | 44 | 1 | 2.3 |
| 40 | 39 | 2 | 5.1 | 44 | 2 | 4.5 |
| 41 | 39 | 0 | 0 | 50 | 3 | 6.0 |
| 42 | 39 | 0 | 0 | 50 | 1 | 2.0 |
| 43 | 39 | 0 | 0 | 50 | 1 | 2.0 |
| 44 | 39 | 3 | 7.7 | 50 | 1 | 2.0 |
| 45 | 39 | 1 | 2.6 | 50 | 1 | 2.0 |
| 46 | 39 | 0 | 0 | 50 | 0 | 0 |
| 47 | 39 | 0 | 0 | 50 | 0 | 0 |
| 48 | 39 | 0 | 0 | 50 | 0 | 0 |
| 49 | 39 | 0 | 0 | 50 | 0 | 0 |
| 50 | 31 | 0 | 0 | 50 | 0 | 0 |
| 51 | 31 | 0 | 0 | 50 | 1 | 2.0 |
| 52 | 31 | 0 | 0 | 43 | 0 | 0 |
| 53 | 22 | 0 | 0 | 24 | 0 | 0 |
| 54 | 13 | 0 | 0 | 23 | 0 | 0 |
| 55 | 8 | 0 | 0 | 23 | 0 | 0 |
| 56 | 8 | 0 | 0 | 14 | 0 | 0 |
| 57 | 3 | 0 | 0 | 14 | 0 | 0 |
| 58 | 3 | 0 | 0 | 6 | 0 | 0 |
| 59 | 3 | 0 | 0 | 6 | 0 | 0 |
| 60 | 3 | 0 | 0 | 6 | 0 | 0 |
| 61 | 3 | 0 | 0 | 6 | 0 | 0 |
| 62 | | | | 6 | 0 | 0 |
| 63 | | | | 6 | 0 | 0 |

Mild diarrhoea was observed in the individuals in the control group on days 33, 40, 44 and 45 and affected 7%, 5%, 8% and 3% of the piglets.

**Table 27**

| The occurrence of medium severe diarrhoea in the post-weaning phase | | | | | | |
|---|---|---|---|---|---|---|
| | **Control** | | | **Experiment** | | |
| | Mild | | | Mild | | |
| Age | Piglet no | Diarrhoea (no) | Control % | Piglet no | Diarrhoea (no) | Experiment % |
| 27 | 8 | 0 | 0 | | | |
| 28 | 8 | 0 | 0 | | | |
| 29 | 8 | 0 | 0 | 7 | 0 | 0 |
| 30 | 17 | 0 | 0 | 26 | 0 | 0 |
| 31 | 26 | 0 | 0 | 27 | 0 | 0 |
| 32 | 31 | 2 | 6.5 | 27 | 2 | 7.4 |
| 33 | 31 | 0 | 0 | 36 | 1 | 2.8 |
| 34 | 36 | 0 | 0 | 36 | 1 | 2.8 |
| 35 | 36 | 0 | 0 | 44 | 2 | 4.5 |
| 36 | 36 | 0 | 0 | 44 | 2 | 4.5 |
| 37 | 36 | 1 | 2.8 | 44 | 3 | 6.8 |
| 38 | 36 | 3 | 8.3 | 44 | 1 | 2.3 |
| 39 | 39 | 0 | 0 | 44 | 1 | 2.3 |
| 40 | 39 | 0 | 0 | 44 | 1 | 2.3 |
| 41 | 39 | 1 | 2.6 | 50 | 1 | 2.0 |
| 42 | 39 | 3 | 7.7 | 50 | 2 | 4.0 |
| 43 | 39 | 1 | 2.6 | 50 | 2 | 4.0 |
| 44 | 39 | 0 | 0 | 50 | 0 | 0 |
| 45 | 39 | 0 | 0 | 50 | 0 | 0 |
| 46 | 39 | 0 | 0 | 50 | 1 | 2.0 |
| 47 | 39 | 0 | 0 | 50 | 0 | 0 |
| 48 | 39 | 0 | 0 | 50 | 1 | 2.0 |
| 49 | 39 | 0 | 0 | 50 | 0 | 0 |
| 50 | 31 | 0 | 0 | 50 | 0 | 0 |
| 51 | 31 | 0 | 0 | 50 | 0 | 0 |
| 52 | 31 | 0 | 0 | 43 | 0 | 0 |
| 53 | 22 | 0 | 0 | 24 | 0 | 0 |
| 54 | 13 | 0 | 0 | 23 | 0 | 0 |
| 55 | 8 | 0 | 0 | 23 | 0 | 0 |
| 56 | 8 | 0 | 0 | 14 | 0 | 0 |
| 57 | 3 | 0 | 0 | 14 | 0 | 0 |
| 58 | 3 | 0 | 0 | 6 | 0 | 0 |
| 59 | 3 | 0 | 0 | 6 | 0 | 0 |
| 60 | 3 | 0 | 0 | 6 | 0 | 0 |
| 61 | 3 | 0 | 0 | 6 | 0 | 0 |
| 62 | | | | 6 | 0 | 0 |
| 63 | | | | 6 | 0 | 0 |

The occurrence ratio of medium severe diarrhoea was also similarly scattered in the group. On day 32 2 piglets suffered symptoms, i.e. 3% of the animals. Following this diarrhoea occurs on days 37-43. On day 37 3% of the animals had symptoms while on day 38 this figure was 8%. In the control population diarrhoea occurred on days 41, 42 and 43 affecting 3%, 8% and 3% of the animals.

At the age of 32-45 days the occurrence ratio projected onto one day in the case of the experiment animals was similar to that of the control animals (2-8%).

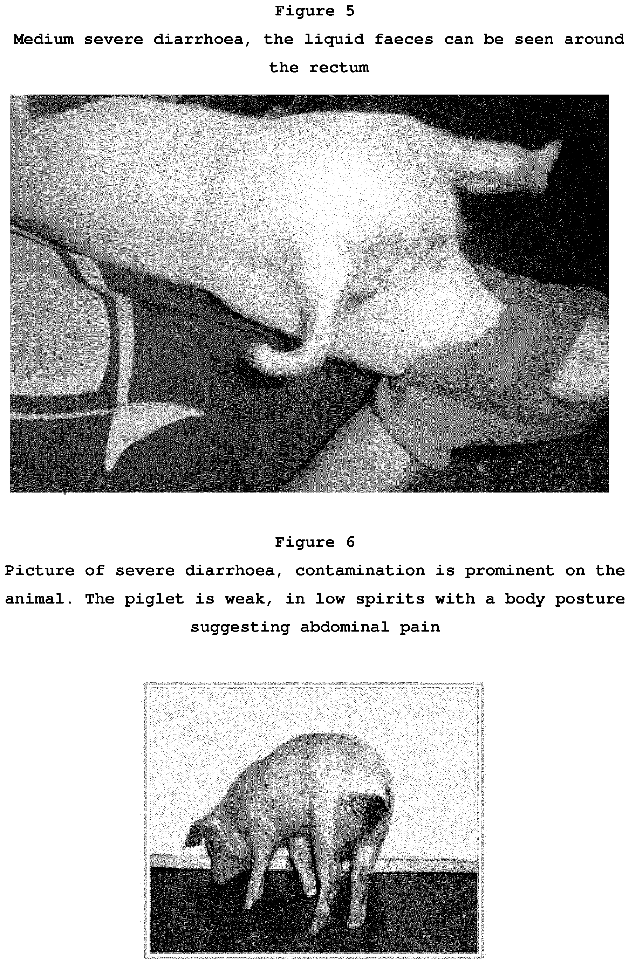

Images of mild, medium severe and severe diarrhoea are presented in Figure 2-6 on drawings 2 and 3.

## Claims

1. Feed supplement preparation containing humic acids, zinc and copper compounds for use in the prevention and/or supplementary treatment of diarrhoea diseases in suckling and newly weaned piglets, wherein the mass ratio of the humic acids (HS), the zinc (Zn) and copper (Cu) calculated using elemental zinc and copper is between 100(HS):2-10 (Zn): 1-10 (Cu), preferably 100(HS):2-6 (Zn): 2.5-7 (Cu), and most preferably 100(HS):2.5-5 (Zn): 3-6 (Cu).

2. Preparation according to claim 1, **characterised by** that the diarrhoea disease is coli-enteritis, Transmissible Gastroenteritis, porcine reproductive and respiratory syndrome (PRRS), coccidiosis, post weaning multisystemic wasting disease, and neonatal piglet disease (NNPD).

3. Preparation according to claim 1 or 2, **characterised by** that the cause of the diarrhoea disease is physiological stress and/or pathogenic microorganisms, preferably bacterias or viruses.

4. Preparation according to any of claims 1-3, **characterised by** that the cause of the diarrhoea disease is Escherichia coli, Clostridium perfringens A, Clostridium perfringens C, Clostridium difficile, Enterococcus durans, Transmissible Gastroenteritis (TGE), rotaviruses, coronaviruses, Isospora suis, Clostridium spp., the Porcine Epidemic Diarrhoea (PED) virus, Porcine Circovirus 2 (PCV2), bacteria or other microorganisms.

5. Preparation according to any of claims 1-4, **characterised by** that as the source of humic acid it contains fulvic acid, peat or lignite, preferably fulvic acid or a mixture of these, more preferably fulvic acid, peat containing at least 40% humic acid or a mixture of these, as the source of zinc organic or inorganic zinc salts, complexes, the hydrates of these or zinc dioxide, as an organic salt or complex preferably zinc humate, zinc acetate, zinc citrate, as an inorganic salt zinc sulphate, zinc chloride, preferably zinc sulphate monohydrate, as the source of copper organic or inorganic copper salts, complexes, or the hydrates of these, preferably copper sulphate.

6. Preparation according to any of claims 1-5, **characterised by** that the pharmacological dose of the zinc used is 60-800 ppm, preferably 60-260 ppm, more preferably 90-190 ppm, and most preferably 100-160 ppm.

7. Feed supplement additive that contains humic acids, zinc and copper compounds, wherein the mass ratio of the humic acids (HS), the zinc (Zn) and copper (Cu) calculated using elemental zinc and copper is between 100(HS):2-10 (Zn): 1-10 (Cu), preferably 100(HS):2-6 (Zn): 2.5-7 (Cu), and most preferably 100(HS) :2.5-5 (Zn): 3-6 (Cu).

8. Feed supplement additive according to claim 7, **characterised by** that as the source of humic acid it contains fulvic acid, peat or lignite, preferably fulvic acid or a mixture of these, more preferably fulvic acid, peat containing at least 40% humic acid or a mixture of these, as the source of zinc organic or inorganic zinc salts, complexes, the hydrates of these or zinc dioxide, as an organic salt or complex preferably zinc humate, zinc acetate, zinc citrate, as an inorganic salt zinc sulphate, zinc chloride, preferably zinc sulphate monohydrate, as the source of copper organic or inorganic copper salts, complexes, or the hydrates of these, preferably copper sulphate.

9. Feed supplement additive according to any of claims 7-8, **characterised by** that the preparation also contains further auxiliary materials apart from the humic acids, the zinc and the copper.

10. Feed supplement additive according to claim 7, **characterised by** that the feed supplement mixture contains humic acids, zinc and copper compounds and in which the mass ratio of the humic acids, the zinc and copper, calculated for elemental zinc and copper, is between 100 (HS) :2-10 (Zn): 1-10 (Cu), preferably 100(HS):2-6 (Zn): 2.5-7 (Cu), most preferably 100(HS):2.5-5 (Zn): 3-6 (Cu), and the zinc concentration in the mixture is 60-800 ppm, preferably 60-260 ppm, more preferably 90-190 ppm, most preferably 100-160 ppm.

11. Feed supplement additive according to claim 10, **characterised by** that as the source of humic acid it contains fulvic acid, peat or lignite, preferably fulvic acid or a mixture of these, more preferably fulvic acid, peat containing at least 40% humic acid or a mixture of these, as the source of zinc organic or inorganic zinc salts, complexes, the hydrates of these or zinc dioxide, as an organic salt or complex preferably zinc humate, zinc acetate, zinc citrate, as an inorganic salt zinc sulphate, zinc chloride, preferably zinc sulphate monohydrate, as the source of copper organic or inorganic copper salts, complexes, or the hydrates of these, preferably copper sulphate.

12. Method for the production of a feed supplement preparation containing humic acids, zinc and copper, **characterised by** that the humic acids, or materials containing humic acids, preferably peat or lignite are mixed with copper and zinc compounds and, optinally, other auxiliary materials, wherein the mass ratio of the humic acids (HS), the zinc (Zn) and copper (Cu) calculated using elemental zinc and copper is between 100(HS):2-10 (Zn): 1-10 (Cu), preferably 100(HS):2-6 (Zn): 2.5-7 (Cu), and most preferably 100(HS):2.5-5 (Zn): 3-6 (Cu).

13. Method according to claim 12, **characterised by** that as the source of humic acid, copper and zinc it uses any material used in livestock breeding containing humic acid, copper and zinc compounds, as the source of humic acid it contains fulvic acid, peat or lignite, preferably fulvic acid or a mixture of these, more preferably fulvic acid, peat containing at least 40% humic acid or a mixture of these, as the source of zinc organic or inorganic zinc salts, complexes, the hydrates of these or zinc dioxide, as an organic salt or complex preferably zinc humate, zinc acetate, zinc citrate, as an inorganic salt zinc sulphate, zinc chloride, preferably zinc sulphate monohydrate, as the source of copper organic or inorganic copper salts, complexes, or the hydrates of these, preferably copper sulphate.

## Patentansprüche

1. Ergänzungsfuttermittelzubereitung umfassend Huminsäuren, Zink- und Kupferverbindungen zur Verwendung bei der Vorbeugung und/oder ergänzenden Behandlung von Durchfallerkrankungen bei säugenden und neu abgesetzten Ferkeln, wobei das Massenverhältnis der Huminsäuren (HS), des Zinks (Zn) und des Kupfers (Cu), berechnet unter Verwendung von elementarem Zink und Kupfer, zwischen 100(HS):2-10 (Zn):1-10 (Cu), bevorzugt 100(HS):2-6 (Zn):2.5-7 (Cu), und am bevorzugtesten 100(HS):2,5-5 (Zn): 3-6 (Cu) beträgt.

2. Zubereitung nach Anspruch 1, **dadurch gekennzeichnet, dass** die Durchfallerkrankung Coli-Enteritis, Transmissible Gastroenteritis, Seuchenhafter Spätabort von Schweinen (*Porcine reproductive and respiratory syndrome* (PRRS)), Kokzidiose, seuchenhaftes Kümmern nach dem Absetzen (*post weaning multisystemic wasting disease*) und die Neugeborenen-Ferkel-Krankheit (NNPD) ist.

3. Zubereitung nach Anspruch 1 oder 2, **dadurch gekennzeichnet, dass** die Ursache der Durchfallerkrankung physiologischer Stress und/oder pathogene Mikroorganismen, bevorzugterweise Bakterien oder Viren, sind.

4. Zubereitung nach einem der Ansprüche 1-3, **dadurch gekennzeichnet, dass** die Ursache der Durchfallerkrankung Escherichia coli, Clostridium perfringens A, Clostridium perfringens C, Clostridium difficile, Enterococcus durans, Transmissible Gastroenteritis (TGE), Rotaviren, Coronaviren, Isospora suis, Clostridium spp, das Porcine Epidemische Diarrhoe (PED)-Virus, Porcine Circovirus 2 (PCV2), Bakterien oder andere Mikroorganismen sind.

5. Zubereitung nach einem der Ansprüche 1-4, **dadurch gekennzeichnet, dass** als Quelle für Huminsäuren Fulvinsäure, Torf oder Braunkohle, bevorzugt Fulvinsäure oder eine Mischung aus diesen, bevorzugter Fulvinsäure, Torf mit mindestens 40% Huminsäuren oder eine Mischung aus diesen, als Quelle für zinkorganische oder anorganische Zinksalze Komplexe, deren Hydrate oder Zinkdioxid, als organisches Salz oder Komplex bevorzugterweise Zinkhumat, Zinkacetat, Zinkcitrat, als anorganisches Salz Zinksulfat, Zinkchlorid, bevorzugterweise Zinksulfatmonohydrat, als Quelle für kupferorganische oder anorganische Kupfersalze Komplexe oder deren Hydrate, bevorzugterweise Kupfersulfat umfasst sind.

6. Zubereitung nach einem der Ansprüche 1-5, **dadurch gekennzeichnet, daß** die pharmakologische Dosis des verwendeten Zinks 60-800 ppm, bevorzugterweise 60-260 ppm, bevorzugter 90-190 ppm und am bevorzugtesten 100-160 ppm beträgt.

7. Ergänzungsfuttermitteladditiv, das Huminsäuren, Zink- und Kupferverbindungen umfasst, wobei das Massenverhältnis der Huminsäuren (HS), des Zinks (Zn) und des Kupfers (Cu), berechnet unter Verwendung von elementarem Zink und Kupfer, zwischen 100(HS):2-10 (Zn): 1-10 (Cu), bevorzugterweise 100(HS):2-6 (Zn): 2,5-7 (Cu), und am bevorzugtesten 100(HS):2,5-5 (Zn): 3-6 (Cu) beträgt.

8. Ergänzungsfuttermitteladditiv nach Anspruch 7, **dadurch gekennzeichnet, dass** als Quelle für Huminsäuren Fulvinsäure, Torf oder Braunkohle, bevorzugterweise Fulvinsäure oder eine Mischung aus diesen, bevorzugter Fulvinsäure, Torf mit mindestens 40% Huminsäuren oder eine Mischung aus diesen, als Quelle für zinkorganische oder anorganische Zinksalze Komplexe, deren Hydrate oder Zinkdioxid, als organisches Salz oder Komplex bevorzugterweise Zinkhumat, Zinkacetat, Zinkcitrat, als anorganisches Salz Zinksulfat, Zinkchlorid, bevorzugterweise Zinksulfatmonohydrat, als Quelle für kupferorganische oder anorganische Kupfersalze Komplexe oder deren Hydrate, bevorzugterweise Kupfersulfat umfasst sind.

9. Ergänzungsfuttermitteladditiv nach einem der Ansprüche 7-8, **dadurch gekennzeichnet, dass** die Zubereitung zusätzlich zu den Huminsäuren, dem Zink und dem Kupfer noch weitere Hilfsstoffe umfasst.

10. Ergänzungsfuttermitteladditiv nach Anspruch 7, **dadurch gekennzeichnet, dass** die Ergänzungsfuttermittelmischung Huminsäuren, Zink- und Kupferverbindungen umfasst und wobei das Massenverhältnis der Huminsäuren, des Zinks und des Kupfers, berechnet für elementares Zink und Kupfer, zwischen 100(HS):2-10 (Zn): 1-10 (Cu), bevorzugterweise 100(HS):2-6 (Zn): 2.5-7 (Cu), am bevorzugtesten 100(HS):2,5-5 (Zn): 3-6 (Cu) beträgt, und die Zinkkonzentration in der Mischung 60-800 ppm, bevorzugterweise 60-260 ppm, bevorzugter 90-190 ppm, am bevorzugtesten 100-160 ppm, beträgt.

11. Ergänzungsfuttermitteladditiv nach Anspruch 10, **dadurch gekennzeichnet, dass** als Quelle für Huminsäuren Fulvinsäure, Torf oder Braunkohle, bevorzugterweise Fulvinsäure oder eine Mischung aus diesen, bevorzugter Fulvinsäure, Torf mit mindestens 40% Huminsäuren oder eine Mischung aus diesen, als Quelle für zinkorganische oder anorganische Zinksalze Komplexe, deren Hydrate oder Zinkdioxid, als organisches Salz oder Komplex bevorzugterweise Zinkhumat, Zinkacetat, Zinkcitrat, als anorganisches Salz Zinksulfat, Zinkchlorid, bevorzugterweise Zinksulfatmonohydrat, als Quelle für kupferorganische oder anorganische Kupfersalze Komplexe oder deren Hydrate, bevorzugterweise Kupfersulfat umfasst sind.

12. Verfahren zur Herstellung einer Ergänzungsfuttermittelzubereitung, die Huminsäuren, Zink und Kupfer umfasst, **dadurch gekennzeichnet, dass** die Huminsäuren oder Materialien, die Huminsäuren umfassen, vorzugsweise Torf oder Braunkohle mit Kupfer- und Zinkverbindungen und optional anderen Hilfsstoffen gemischt werden, wobei das Massenverhältnis der Huminsäuren (HS), des Zinks (Zn) und des Kupfers (Cu), berechnet unter Verwendung von elementarem Zink und Kupfer, zwischen 100(HS):2-10 (Zn): 1-10 (Cu), bevorzugterweise 100(HS):2-6 (Zn): 2,5-7 (Cu), und am bevorzugtesten 100(HS):2,5-5 (Zn): 3-6 (Cu) beträgt.

13. Verfahren nach Anspruch 12, **dadurch gekennzeichnet, dass** als Quelle für Huminsäuren, Kupfer und Zink jedes in der Tierzucht verwendete Material verwendet wird, das Huminsäuren, Kupfer- und Zinkverbindungen umfasst, als Quelle für Huminsäuren Fulvinsäure, Torf oder Braunkohle, bevorzugterweise Fulvinsäure oder eine Mischung aus diesen, bevorzugter Fulvinsäure, Torf mit mindestens 40% Huminsäuren oder eine Mischung aus diesen, als Quelle für zinkorganische oder anorganische Zinksalze Komplexe, deren Hydrate oder Zinkdioxid, als organisches Salz oder Komplex bevorzugterweise Zinkhumat, Zinkacetat, Zinkcitrat, als anorganisches Salz Zinksulfat, Zinkchlorid, bevorzugterweise Zinksulfatmonohydrat, als Quelle für kupferorganische oder anorganische Kupfersalze Komplexe oder deren Hydrate, bevorzugterweise Kupfersulfat umfasst sind.

## Revendications

1. Préparation de compléments alimentaires contenant des acides humiques, des composés du zinc et du cuivre pour l'utilisation dans la prévention et/ou le traitement avec des compléments des maladies diarrhéiques des porcelets allaités et nouvellement sevrés, dans laquelle le rapport de masse entre les acides humiques (HS), le zinc (Zn) et le cuivre (Cu) calculé en utilisant le zinc et le cuivre élémentaires est compris entre 100(HS):2-10 (Zn): 1-10 (Cu), préférablement 100(HS):2-6 (Zn): 2,5-7 (Cu), et le plus préférablement 100(HS):2,5-5 (Zn): 3-6 (Cu) .

2. Préparation selon la revendication 1, **caractérisée en ce que** la maladie de la diarrhée est entérite à E. coli, gastro-entérite transmissible, syndrome respiratoire et reproducteur porcin (SRRP), coccidiose, syndrome de dépérissement post-sevrage multisystémique, et la maladie néonatale des porcelets (NNPD).

3. Préparation selon la revendication 1 ou 2, **caractérisée en ce que** la cause de la maladie de la diarrhée est un stress physiologique et/ou des microorganismes pathogènes, de préférence bactéries ou virus.

4. Préparation selon l'une quelconque des revendications 1 à 3, **caractérisée en ce que** la cause de la maladie de la diarrhée est Escherichia coli, Clostridium perfringens A, Clostridium perfringens C, Clostridium difficile, Enterococcus durans, gastro-entérite transmissible (GET), rotavirus, coronavirus, Isospora suis, Clostridium spp., le virus de la diarrhée épidémique porcine (DEP), le circovirus type 2 des porcs (PCV2), bactéries ou autres microorganismes.

5. Préparation selon l'une quelconque des revendications 1 à 4, **caractérisée en ce que** comme source d'acide humique, elle contient de l'acide fulvique, de la tourbe ou du lignite, préférablement de l'acide fulvique ou un mélange de ceux-ci, plus préférablement de l'acide fulvique, la tourbe contenant au moins 40% d'acide humique ou un mélange de ceux-ci, comme source de zinc des sels de zinc organiques ou non organiques, des complexes, les hydrates de ceux-ci ou du dioxyde de zinc, comme sel ou complexe organique préférablement de l'humate de zinc, de l'acétate de zinc, du citrate de zinc, comme sel non organique du sulfate de zinc, du chlorure de zinc, préférablement du monohydrate de sulfate de zinc, comme source de cuivre des sels de cuivre organiques ou non organiques, des complexes, ou les hydrates de ceux-ci, préférablement du sulfate de cuivre.

6. Préparation selon l'une quelconque des revendications 1 à 5, **caractérisée en ce que** la dose pharmacologique du zinc utilisée est 60-800 ppm, préférablement 60-260 ppm, plus préférablement 90-190 ppm, et le plus préférablement 100-160 ppm.

7. Addictif de compléments alimentaires qui contient des acides humiques, des composés du zinc et du cuivre, dans lequel le rapport de masse entre les acides humiques (HS), le zinc (Zn) et le cuivre (Cu) calculé en utilisant du zinc et cuivre élémentaires est entre 100(HS):2-10 (Zn): 1-10 (Cu), préférablement 100(HS):2-6 (Zn): 2,5-7 (Cu), et le plus préférablement 100(HS):2,5-5 (Zn): 3-6 (Cu).

8. Addictif de compléments alimentaires selon la revendication 7, **caractérisé en ce que**, comme source d'acide humique il contient de l'acide fulvique, de la tourbe ou du lignite, préférablement de l'acide fulvique ou un mélange de ceux-ci, plus préférablement de l'acide fulvique, la tourbe contenant au moins 40% d'acide humique ou un mélange de ceux-ci, comme source de zinc des sels de zinc organiques ou non organiques, des complexes, les hydrates de ceux-ci ou du dioxyde de zinc, comme sel ou complexe organique préférablement de l'humate de zinc, de l'acétate de zinc, du citrate de zinc, comme sel non organique du sulfate de zinc, du chlorure de zinc, préférablement du monohydrate de sulfate de zinc, comme source de cuivre des sels de cuivre organiques ou non organiques, des complexes, ou les hydrates de ceux-ci, préférablement du sulfate de cuivre.

9. Addictif de compléments alimentaires selon l'une quelconque des revendications 7 à 8, **caractérisé en ce que** la préparation contient de même, en outre, des matières auxiliaires, autres que celles des acides humiques, du zinc et du cuivre.

10. Addictif de compléments alimentaires selon la revendication 7, **caractérisé en ce que** le mélange de compléments alimentaires contient des acides humiques, des composés du zinc et du cuivre et dans lequel le rapport de masse entre les acides humiques, le zinc et le cuivre, calculé pour le zinc et le cuivre élémentaires, est entre 100(HS):2-10 (Zn): 1-10 (Cu), préférablement 100(HS):2-6 (Zn): 2,5-7 (Cu), le plus préférablement 100(HS):2,5-5 (Zn): 3-6 (Cu), et la concentration de zinc dans le mélange est 60-800 ppm, préférablement 60-260 ppm, plus préférablement 90-190 ppm, le plus préférablement 100-160 ppm.

11. Addictif de compléments alimentaires selon la revendication 10, **caractérisé en ce que**, comme source d'acide humique il contient de l'acide fulvique, de la tourbe ou du lignite, préférablement de l'acide fulvique ou un mélange de ceux-ci, plus préférablement de l'acide fulvique, la tourbe contenant au moins 40% d'acide humique ou un mélange de ceux-ci, comme source de zinc des sels de zinc organiques ou non organiques, des complexes, les hydrates de ceux-ci ou du dioxyde de zinc, comme sel ou complexe organique préférablement de l'humate de zinc, de l'acétate de zinc, du citrate de zinc, comme sel non organique du sulfate de zinc, du chlorure de zinc, préférablement du monohydrate de sulfate de zinc, comme source de cuivre des sels de cuivre organiques ou non organiques, des complexes, ou les hydrates de ceux-ci, préférablement du sulfate de cuivre.

12. Procédé pour la production d'une préparation de compléments alimentaires contenant des acides humiques, du zinc et du cuivre, **caractérisé en ce que** les acides humiques, ou les matières contenant des acides humiques, préférablement tourbe ou lignite, sont mélangés avec des composés du cuivre et du zinc et, facultativement, d'autres matières auxiliaires, dans lequel le rapport de masse entre les acides humiques (HS), le zinc (Zn) et le cuivre (Cu) calculé en utilisant le zinc et le cuivre élémentaires est entre 100(HS):2-10 (Zn): 1-10 (Cu), préférablement 100(HS):2-6 (Zn): 2,5-7 (Cu), et le plus préférablement 100(HS):2,5-5 (Zn): 3-6 (Cu).

13. Procédé selon la revendication 12, **caractérisé en ce que**, comme source d'acide humique, de cuivre et de zinc, il utilise une matière quelconque utilisée dans l'élevage du bétail contenant de l'acide humique, des composés du cuivre et du zinc, comme source d'acide humique il contient de l'acide fulvique, de la tourbe ou du lignite, préférablement de l'acide fulvique ou un mélange de ceux-ci, plus préférablement de l'acide fulvique, la tourbe contenant au moins 40% d'acide humique ou un mélange de ceux-ci, comme source de zinc des sels de zinc organiques ou non organiques, des complexes, les hydrates de ceux-ci ou du dioxyde de zinc, comme sel ou complexe organique préférablement de l'humate de zinc, de l'acétate de zinc, du citrate de zinc, comme sel non organique du sulfate de zinc, du chlorure de zinc, préférablement du monohydrate de sulfate de zinc, comme source de cuivre des sels de cuivre organiques ou non organiques, des complexes, ou les hydrates de ceux-ci, préférablement du sulfate de cuivre.
